# EUROPEAN PATENT SPECIFICATION

(11) **EP 1 572 211 B1**
(45) Date of publication and mention of the grant of the patent: **20.02.2008**
(21) Application number: 03768023.8
(22) Date of filing: 18.12.2003
(51) Int. Cl.: A61K 31/506, A61P 5/48

(54) **THERAPEUTIC APPLICATIONS OF 2-SUBSTITUTED 4-HETEROARYLPYRIMIDINES**
PHARMAZEUTISCHE VERWENDUNGEN VON 2-SUBSTITUIERTEN 4-HETEROARYLPYRIMIDINEN
APPLICATIONS THERAPEUTIQUES DES 4-HETEROARYLRYRIMIDINES 2-SUBSTITUEES

(30) Priority: 19.12.2002 GB 0229581
(43) Date of publication of application: 14.09.2005
(73) Proprietor: Cyclacel Limited, London N1 7JQ (GB)
(72) Inventor: WANG, Shudong, Forfar, Angus DD8 2RZ (GB); MEADES, Christopher, Dundee DD4 0TE (GB); WOOD, Gavin, Cupar, Fife KY15 5DP (GB); DUNCAN, Kenneth, Cambusbarron, Stirlingshire FK7 9RA (GB); ZHELEVA, Daniella, Newport on Tay, Fife DD6 8NR (GB); FISCHER, Peter, Arbroath, Angus DD11 2EN (GB)
(74) Representative: Clyde-Watson, Zöe
(86) International application number: PCT/GB2003/005558
(87) International publication number: WO 2004/056368

(56) References cited:
- EP-A- 0 233 461
- WO-A-02/066480
- WO-A-02/096905
- WO-A-03/029248
- US-A- 5 958 935
- US-A1- 2002 019 404
- US-B1- 6 417 185

## Description

The present invention relates to new therapeutic applications of 2-substituted 4-heteroaryl-pyrimidines.

### BACKGROUND TO INVENTION

Certain 4,5,6-substituted-N-(substituted-phenyl)-2-pyrimidineamines having anti-asthmatic properties are disclosed in EP-A-233,461. Certain 4-heteroaryl-N-(3-substituted-phenyl)-2-pyridineamines possessing anti-proliferative properties and inhibiting protein kinase C, epidermal growth factor receptor-associated tyrosine protein kinase (EGF-R-TPK), as well as CDKl/cycli.n B have been disclosed in WO95/09847 wherein the exemplified heteroaryl groups are pyridyl and indolyl.

J. Med. Chem. (1993) Vol. 36, pages 2716-2725, Paul, R. et al discloses a further class of phenyl amino-pyrimidines possessing anti-inflammatory activity. These compounds include mono-substituted 2-thienyl groups at the 4-position of the pyrimidine ring and dimethyl-3-furyl groups at this position.

We have previously disclosed certain anti-proliferative thiazolo- and pyrrolo-anilinopyrimidines (WO 01/072745, Cyclacel Limited; WO 02/079193, Cyclacel Limited). The compounds disclosed therein were surprisingly found not to inhibit protein kinase C.

The present invention relates to previously undisclosed therapeutic applications of 2-substituted 4-heteroaryl-pyrimidines.

### STATEMENT OF INVENTION

A first aspect of the invention relates to the use of a compound of formula I, or a pharmaceutically acceptable salt thereof, 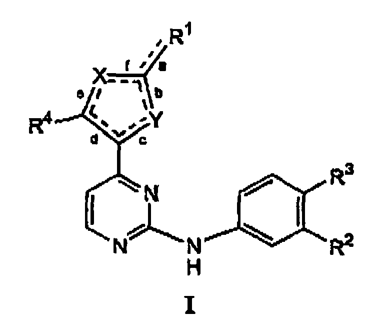 wherein
(A) one of X and Y is S, and the other is N; or
   one of X and Y is NH or N-R⁵, and the other is C-R⁶;
   "a" is a single bond;
   "b", "c", "d", "e" and "f are single or double bonds so as to form a heteroaryl ring;
   R¹ is R⁷with the proviso that R¹ is other than H or Me; or
(B) one of X and Y is S, and the other is NH or N-R⁵;
   "a" and "d" are each double bonds;
   "b", "c", "e" and "f" are each single bonds;
   R¹ is oxo; and
R², R³, R⁴, R⁵, and R⁶ are each independently H or R⁷;
R¹ is a group (CH₂)ₙ-R⁸, wherein n is 0, 1, 2, 3 or 4 and wherein R⁸ is selected from alkyl aryl, heteroaryl, heterocycloalkyl, F, Cl, Br, I, CF₃, NO₂, CN, OR, O-alkyl, O-aryl, O-heteroaryl, O-heterocycloalkyl, CO-alkyl, CO-aryl, CO-heteroaryl, CO-heterocycloalkyl, COO-akyl, NH₂, NH-alkyl, NH-aryl, N(alkyl)₂, NH-heteroaryl, NH-heterocycloalkyl, COOH, CONH₂, CONH-alkyl, CON(alkyl)₂, CONH-aryl, CONH-heteroaryl, CONH-heterocycloalkyl, SO₃H, SO₁-alkyl, SO₂-aryl, SO₂-heteroaryl, SO₂-heterocycloalkyl, SO₂NH₂, SO₂NH-alkyl, SO₂N(alkyl)₂; SO₂NH-aryl, SO₂NH heteroaryl, or SO₂NH-heterocycloalkyl, wherein said alkyl, aryl, heteroaryl, and heterocycloalkyl groups are optionally substituted with one or more groups selected from halogeno, NO₂, OH, O-methyl, NH₂, COOH, CONH₂ and CF₃; in the preparation of a medicament for treating diabetes.

### DETAILED DESCRIPTION

As used herein, the term "alkyl" includes both saturated straight chain and branched alkyl groups which may be substituted (mono- or poly-) or unsubstituted. Preferably, the alkyl group is a C₁₋₂₀ alkyl group, more preferably a C₁₋₁₅, more preferably still a C₁₋₁₂ alkyl group, more preferably still, a C₁₋₆ alkyl group, more preferably a C₁₋₃ alkyl group. Particularly preferred alkyl groups include, for example, methyl, ethyl, propyl, isopropyl, butyl, isobutyl, tert-butyl, pentyl and hexyl.

As used herein, the term "aryl" refers to a substituted (mono- or poly-) or unsubstituted monoaromatic or polyaromatic system, wherein said polyaromatic system may be fused or unfused. Preferably, the term "aryl" is includes groups having from 6 to 10 carbon atoms, e.g. phenyl, naphthyl etc. More preferably, the aryl group contains 6 carbons, for example, a phenyl group. The term "aryl" is synonymous with the term "aromatic".

As used herein, the term "heteroaryl" refers to five- and six-membered aromatic rings containing up to 4 heteroatoms, each independently selected from N, O, and S. Preferred heteroaryl groups include pyrrole, pyrazole, pyrimidine, pyrazine, pyridine, quinoline, thiazole, thiophene and furan.

As used herein, the term "heterocycloalkyl" refers to saturated or unsaturated five- and six-membered cyclic systems containing up to 4 heteroatoms each independently selected from N, O, and S.

In one preferred embodiment, the invention relates to the use of a compound of formula Ia, or a pharmaceutically acceptable salt thereof, 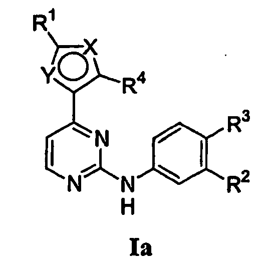 wherein
one of X and Y is N and the other is S; or
one of X and Y is NH orN-R⁵ and the other is C-R⁶;
R¹ is R⁷ with the proviso that R¹ is other than H or Me.
R², R³, R⁴, R⁵, and R⁶ are each independently H or R⁷;
R⁷ is a group (CH₂)ₙ-R⁸, wherein n is 0, 1, 2, 3 or 4 and wherein R⁸ is selected from alkyl, aryl, heteroaryl, heterocycloalkyl, F, Cl, Br, I, CF₃; NO₂, CN, OH, O-alkyl, O-aryl, O-heteroaryl, O-heterocycloalkyl, CO-alkyl, CO-aryl, CO-heteroaryl, CO-heterocycloalkyl, COO-alkyl, NH₂, NH-alkyl, NH-aryl, N(alkyl)₂, NH-heteroaryl, NH-heterocycloalkyl, COOH, CONH₂, CONH-alkyl, CON(alkyl)₂, CONH-aryl, CONH-heteroaryl, CONH-heterocycloallcyl, SO₃H, SO₂-alkyl, SO₂-aryl, SO₂-heteroaryl, SO₂-heterocycloalkyl, SO₂NH₂, SO₂NH-alkyl, SO₂N(alkyl)₂, SO₂NH-aryl, SO₂NH-heteroaryl, or SO₂NH-heterocycloalkyl, wherein said alkyl, aryl, heteroaryl, and heterocycloalkyl groups are optionally substituted with one or more groups selected from halogeno, NO₂, OH, O-methyl, NH₂, COOH, CONH₂ and CF₃;
in the preparation of a medicament for treating diabetes.

Preferred compounds of the invention include those of formula Id, Ie, If, Ig, Ih and Ii shown below.

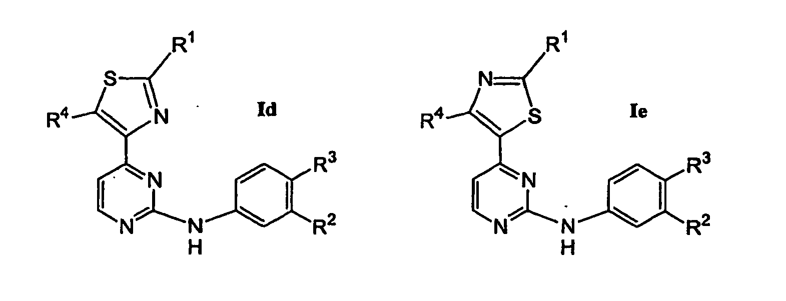

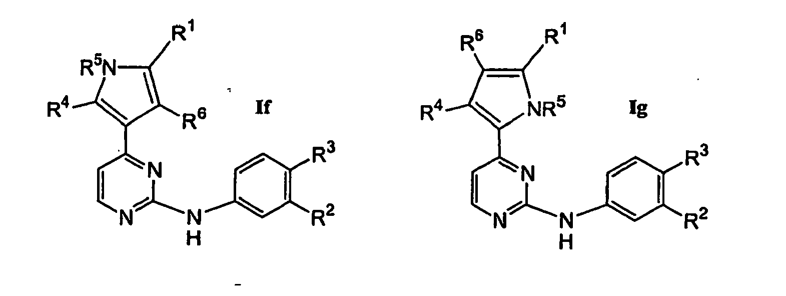

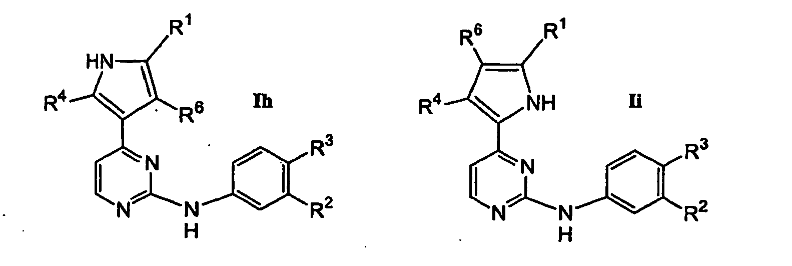

For compounds of formula Ia, in a first preferred embodiment, X is N and Y is S; or X is NH or N-R⁵ and Y is C-R⁶.

Preferably, for said first preferred embodiment:
R¹ is selected from CN, CONH₂, NO₂, halo, CH₂N(alkyl)₂, O-alkyl, NH₂ and NH-alkyl;
R² is selected from NO₂, H, OH, halo and alkyl;
R³ is selected from H, halo, OH, CF₃, alkyl, N(alkyl)₂, O-alkyl, heterocycloalkyl and COO-alkyl;
R⁴ is alkyl;
R⁵ is H or alkyl; and
R⁶ is alkyl.

Even more preferably, for said first preferred embodiment:
R¹ is selected from CN, CONH₂, NO₂, Br, Cl, CH₂NMe₂, OMe, NH₂ and NHMe;
R² is selected from NO₂, H, OH, I, Me, F and Cl;
R³ is selected from H, F, OH, CF₃, I, Me, Cl, NMe₂, OMe, morpholino and COOEt;
R⁴ is Me;
R⁵ is H or Me; and
R⁶ is Me.

For compounds of formula Ia, in a second preferred embodiment, X is NH or N-R⁵ and Y is C-R⁶.

Preferably, in said second preferred embodiment,
R¹ is selected from CN, CONH₂, NO₂, halo and CH₂N(alkyl)₂;
R² is selected from NO₂, H, OH, halo and alkyl;
R³ is selected from H, halo, OH, CF₃, alkyl and N(alkyl)₂;
R⁴ is alkyl;
R⁵ is H or alkyl; and
R⁶ is alkyl.

Even more preferably, in said second preferred embodiment,
R¹ is selected from CN, CONH₂, NO₂, Br, Cl and CH₂NMe₂;
R² is selected from NO₂, H, OH, I, Me and F;
R³ is selected from H, F, OH, CF₃, I, Me, Cl and NMe₂;
R⁴ is Me;
R⁵ is H or Me; and
R⁶ is Me.

More preferably still, in said second preferred embodiment,
R¹ is CN or CONH₂;
R² is NO₂ or H; and
R³ is F or Me.

In a third preferred embodiment of the invention, X is N and Y is S.

For said third preferred embodiment, preferably,
R¹ is selected from halo, NH₂ and NH-alkyl;
R² is selected from NO₂, H, OH, halo and alkyl;
R³ is selected from H, halo, OH, alkyl, N(alkyl)₂, O-alkyl, heterocycloalkyl and COO-alkyl.;
R⁴ is alkyl.

More preferably, in said third preferred embodiment,
R¹ is selected from Cl, NH₂ and NHMe;
R² is selected from NO₂, H, OH, Me and Cl; and
R³ is selected from H, F, OH, Me, Cl, NMe₂, OMe, morpholino and COOEt;
R⁴ is Me.

More preferably still, in said third preferred embodiment,
R² is H or NO₂; and
R³ is Cl or F.

Another alternative preferred embodiment of the invention relates to the use of a compound of formula Ib, or a pharmaceutically acceptable salt thereof, 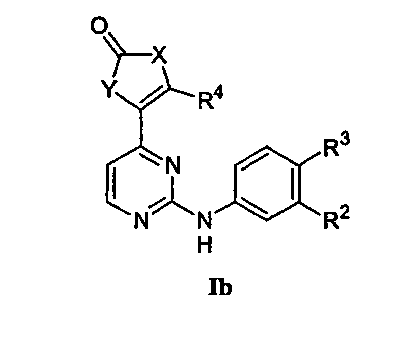 wherein one of X and Y is S, and the other is NH or N-R⁵; and
R²⁻⁵ are as defined above for formula I;
in the preparation of a medicament for treating diabetes.

In one particularly preferred embodiment, Y is S and X is NH or NR⁵.

Preferably, for compounds of formula Ib:
R² is selected from H, OH, NO₂ and alkyl;
R³ is selected from H, halogen, alkoxy, alkyl, N-(alkyl)₂ and OH; and
R⁴ and R⁵ are-each independently alkyl.

More preferably, for compounds of formula Ib:
R² is selected from H, OH, NO₂ and Me;
R³ is selected from H, Cl, F, OMe, Me, NMe₂ and OH; and
R⁴ and R⁵ are both Me.

In one especially preferred embodiment of the invention, the compound of formula I is selected from compounds [1]-[26] and [28]-[39] listed below.

Another aspect of the invention relates to the use of one or more of the following compounds in the preparation of a medicament for the treatment of a GSK-dependent disorder:
3,5-Dimethyl-4-[2-(3-nitro-phenylamino)-pyrimidin-4-yl]-1H-pyrrole-2-carbonitrile **[1];**
4-[2-(4-Fluoro-phenylamino)-pyrimidin-4-yl]-3,5-dimethyl-1H-pyrrole-2-carbonitrile **[2];**
4-[2-(4-Hydroxy-phenylamino)-pyrimidin-4-yl]-3,5-dimethyl-1H-pyrrole-2-carbonitrile **[3];**
3,5-Dimethyl-4-[2-(4-trifluoromethyl-phenylamino)-pyrimidin-4-yl]-1H-pyrrole-2-cathonitrile **[4];**
4-[2-(4-lodo-phenylamino)-pyrimidin-4-yl]-3,5-dimethyl-1H-pyrrole-2-carbonitrile **[5];**
4-[2-(3-Hydroxy-phenylamino)-pyrimidin-4-yl]-3,5-dimethyl-1H-pyrrole-2-carbonitrile **[6];**
3,5-Dimethyl-4-[2-(4-methyl-3-nitro-phenylamino)-pyrimidin-4-yl]-1H-pyrrole-2-carbonitrile **[7];**
4-[2-(3-Iodo-4-methyl-phenylamino)-pyrimidin-4-yl-3,5-dimethyl-1H pyrrole-2-carbonitrile **[8];**
4-[2-(4-Chloro-3-methyl-phenylamino)-pyrimidin-4-yl]-3,5-dimethyl-1H-pyrrole-2-carbonitrile **[9];**
4-[2-(3-Hydroxy-4-methyl-phenylamino)-pyrimidin-4-yl]-3,5-dimethyl-1*H* pyrrole-2-carbonitrile **[10];**
4-[2-(4-Fluoro-3-methyl-phenylamino)-pyrimidin-4-yl]-3,5-dimethyl-1*H*-pyrrole-2-carbonitrile **[11];**
4-[2-(4-Dimethylamino-phenylamino)-pyrimidin-4-yl]-3,5-dimethyl-1*H*- pyrrole-2-carbonitrile **[12];**
4-[2-(4-Fluoro-phenylamino)-pyrimidin-4-yl]-3,5-dimethyl-1H-pyrrole-2-carboxylic acid amide **[13];**
[4-(2,4-Dimethyl-5-nitro-1H-pyrrol-3-yl)-pyrimidin-2-yl]-(4-fluoro-phenyl)-amine **[14];**
N-[4-(2,4-Dimethyl-5-nitro-1H-pyrrol-3-yl)-pyrimidin-2-yl]-N',N'-dimethyl-benzene-1,4-diamine **[15];**
[4-(5-Bromo-2,4-dimethyl-1H-pyrrol-3-yl)-pyrimidin-2-yl]-(4-fluoro-phenyl)-amine **[16]** ;
[4-(5- Bromo-2,4-dimethyl-1H -pyrrol- 3-yl)-pyrimidin-2-yl]-(3 -nitro-phenyl)-amine **[17];**
[4-(5-Chloro-2,4-dimethyl-1H-pyrrol-3-yl)-pyrimidin-2-yl]-(4-fluoro-phenyl)-amine **[18]**;
[4-(5-Dimethylaminomethyl-2,4-dimethyl-1H-pyrrol-3-yl)-pyrimidin-2-yl]-(4-fluorophenyl)-amine **[19];**
4-[2-(4-Dimethylamino-3-nitro-phenylamino)-pyrimidin-4-yl]-3,5-dimethyl-1H-pyrrole-2-carbonitrile **[20];**
N⁴-[4-(2,4-Dimethyl-5-nitro-1H-pyrrol-3-yl)-pyrimidin-2-yl]-N¹,N¹-dimethyl-2-nitrobenzene-1,4-diamine **[21];**
4-[2-(3-Fluoro-4-methyl-phenylamino)-pyrimidin-4-yl]-3,5-dimethyl-1H-pyrrole-2-carbonitrile **[22];**
5-[2-(4-Chloro-phenylamino)-pyrimidin-4-yl]-3,4-dimethyl-3H-thiazol-2-one **[23];**
5-[2-(4-Methoxy-phenylamino)-pyrimidin-4-yl]-3,4-dimethyl-3H-thiazol-2-one **[24];**
S-[2-(3-Hydroxy-phenylamino)-pyrimidin-4-yl]-3,4-dimethyl-3H-thiazol-2-one **[25];**
5-[2-(4- Dimethylamino-phenylamino )-pyrimidin-4-yl]-3,4-dimethyl-3H -thiazol-2-one **[26];**
5-[2-(4-Fluoro-3-nitro-phenylamino)-pyrimidin-4-yl]-3,4-dimethyl-3H-thiazol-2-one **[28];**
2-Chloro-4-[4-(4-methyl-2-methylatnino-thiazol-5-yl)-pyrimidin-2-ylamino]-benzoic acid ethyl ester **[29];**
[4-(2-Amino-4-methyl-thiazol-5-yl)-pyrimidin-2-yl]-(3-nitro-phenyl)-amine **[30];**
3,4-Dimethyl-5-[2-(3-nitro-phenylamino)-pyrimidin-4-yl]-3H-thiazol-2-one **[32];**
5-[2-(4-Fluoro-phenylamino)-pyrimidin-4-yl]-3,4-dimethyl-3H-thiazol-2-one **[33];**
5-[2-(4-Hydroxy-phenylamino)-pyrimidin-4-yl]-3,4-dimethyl-3H-thiazol-2-one **[34];**
[4-(2-Chloro-4-methyl-thiazol-5-yl)-pyrimidin-2-yl]-(3-nitro-phenyl)-amine **[35];**
3,4-Dimethyl-5-[2-(4-methyl-3-nit<o-phenylamino)-pyrimidin-4-yl]-3H-thiazol-2-one **[36];**
5-[2-(4-Fluoro-3-methyl-phenylamino)-pyrimidin-4-yl]-3,4-dimethyl-3H-thiazol-2-one **[37];**
4-[2-(4-Dimethylamino-phenylamino)-pyrimidin-4-yl]-1,3,5-trimethyl-1H-pyrrole-2-carbonitrile **[38];** and
4-[2-(4-Diinethylamino-3-nitro-phenylamino)-pyrimidin-4-yl]-1,3,5-trimethyl-1H-pyrrole-2-carbonitrile **[39].**

More preferably, said compound of formula I is selected from the following:
3,5-Dimethyl-4-[2-(3-nitro-phenylanvno)-pyrimidin-4-yl]-1H-pyrrole-2-carbonitrile **[1];**
4-[2-(4-Fluoro-phenylamino)-pyrimidin-4-yl]-3,5-dimethyl-1H-pyrrole-2-carbonitrile[2];
4-[2-(3-Hydroxy-phenylamino)-pyrimidin-4-yl]-3,5-dimethyl-1H-pyrrole-2-carbonitrile **[6];**
3,5-Dimethyl-4-[2-(4-methyl-3-nitro-phenylamino)-pyrimidin-4-yl]-1H-pyrrole-2-carbonitrile **[7];**
4-[2-(4-Fluoro-3-methyl-phenylainino)-pyrimidin-4.-yl]-3,5-dimethyl-1*H* pyrrole-2-carbonitrile **[11];**
4-[2-(4-Dimethylamino-phenylamino)-pyrimidin-4-yl]-3,5-dimethyl-1*H*-pyrrole-2-carbonitrile **[12];**
4-[2-(4-Fluoro-phenylamino)-pyrimidin-4-yl]-3,5-dimethyl-1H-pyrrole-2-carboxylic acid amide **[13];**
4-[2-(3-Fluoro-4-methyl-phenylamino)-pyrimidin-4-yl]-3,5-dimethyl-1H-pyrrole-2-carbonitrile **[22];**
(4-Fluoro-phenyl)-[4-(2-methyl-4-phenyl-1H-pyrrol-3-yl)-pyrimidin-2-yl]-amine **[27];**
3,4-Dimethyl-5-[2-(3-nitro-phenylamino)-pyrimidin-4-yl]-3H-thiazol-2-one **[32];**
5-[2-(4-Hydroxy-phenylamino)-pyrimidin-4-yl]-3,4-dimethyl-3H-thiazol-2-one **[34];**
3,4-Dimethyl-5-[2-(4-methyl-3-nitro-phenylamino)-pyrinudin-4-yl]-3H-thiazol-2-one **[36];**
S-[2-(4-Fluoro-3-methyl-phenylami.no)-pyrimidin-4-yl]-3,4-dimethyl-3H-thiazol-2-one **[37];**
4-[2-(4-Dimethylamino-phenylamino)-pynmidin-4-yl] - 1,3,5-trimethyl- 1H-pyrrole-2-carbonitrile **[38];** and
4-[2-(4-Dimethylamino-3-nitro-phenylamino)-pyrimidin-4-yl]-1,3,5-trimethyl-1H-pyrrole-2-carbonitrile **[39].**

Even more preferably, said compound of formula I is selected from the following:
3,5-Dimethyl-4-[2-(4-methyl-3-nitro-phenylamino)-pyiimidin-4-yl]-1H-pyrrole-2-carbonitrile **[7];**
4-[2-(4-Fluoro-phenylamino)-pyrimidin-4-yl]-3,5-dimethyl-1H-pyrrole-2-carboxylic acid amide **[13];**
(4-Fluoro-phenyl)-[4-(2-methyl-4-phenyl-1H-pyrrol-3-yl)-pyrimidin-2-yl]-amine **[27];**
3,4-Dimethyl-5-[2-(3-nitro-phenylamino)-pyrimidin-4-yl]-3H-thiazol-2-one **[32];**
3,4-Dimethyl-5-[2-(4-methyl-3-nitro-phenylamino)-pyrimidin-4-yl]-3H-thiazol-2-one **[36];**
5-[2-(4-Fluoro-3-methyl-phenylamino)-pyrimidin-4-yl]-3,4-dimethyl-3H-thiazol-2-one **[37];**
4-[2-(4-Dimethylamino-phenylamino)-pyrimidin-4-yl]-1 ,3,5-trimethyl-1H -pyrrole-2-carbonitrile **[38];** and
4-[2-(4-Dimethylamino-3-nitro-phenylamino)-pyrimidin-4-yl]-1,3,5-trimethyl-1H-pyrrole-2-carbonitrile **[39].**

Most preferably, said compound of formula I is selected from the following:
4-[2-(4-Dimethylamino-3-nitro-phenylamino)-pyrimidin-4-yl]-3,5-dimethyl-1H-pyrrole-2-carbonitrile **[20];** and
N⁴-[4-(2,4-Dimethyl-5-nitro-1H-pyrrol-3-yl)-pyrimidin-2-yl]-N¹,N¹-dimethyl-2-nitrobenzene-1,4-diamine **[21].**

In one particularly preferred embodiment of the invention, the compound of formula I is capable of inhibiting GSK3β. Preferably, said compound of formula I exhibits an IC₅₀ value of less than 1 µM as measured by a GSK3β kinase assay. Details of a suitable GSK3β kinase assay-are described in the accompanying Examples section. Preferably, said compound is selected from [1]-[3], [6], [7], [10], [11], [13]-[15], [17], [20]-[23] and [25]-[29]. More preferably, said compound of formula I exhibits an IC₅₀ value of less than 0.1 µM. More preferably, the compound is selected from [7], [13], [20], [21], [23], [25], [28], [32], [33], [34], [35], [36], [37] and [39]. Even more preferably, said compound of formula I exhibits an IC₅₀ value less than 0.01 µM. Even more preferably, the compound is selected from [20], [28], [32] and [35]. More preferably still, said compound of formula I exhibits an IC₅₀ value of less than 0.001 µM. More preferably still, the compound is selected from [32] and [35].

In another preferred embodiment, the compound of formula exhibits selectivity for inhibiting GSK3β over CDKs, for example CDK2/E, as measured by the appropriate kinase assays. Preferably, the compound exhibits a selectivity, IC₅₀ (CDK2/E)/(GSK3β), of 2 or more. Preferably, the compound is selected from [7], [13], [20]-[23], [25]-[28], [32], [33] and [35]-[39]. More preferably, the compound exhibits a selectivity, IC₅₀ (CDK2/E)/(GSK3β), of 5 or more. More preferably, the compound is selected from [7], [20], [23], [27], [28], [32], [33], [35], [38] and [39]. More preferably still, the compound exhibits a selectivity, IC₅₀ (CDK2/E)/(GSK3β), of 10 or more. More preferably still, the compound is selected from [7], [20], [23], [27], [28], [32] and [35].

In another preferred embodiment, the compound of formula I is capable of inducing cellular glycogen synthase (GS) activity as measured by an appropriate assay. Preferably, the compound is selected from [1]-[3], [6], [7], [11]-[13], [20]-[22], [27], [32], [34] and [36]-[39]. More preferably, said compound exhibits a 3-fold or more increase in cellular GS activity over control samples. More preferably, the compound is selected from [1], [2], [6], [7], [21], [32], [36], [37] and [39].

### THERAPEUTIC APPLICATIONS

As used herein the phrase "preparation of a medicament" includes the use of compounds of formula I directly as the medicament in addition to their use in a screening programme for identifying further therapeutic agents or in any stage of the manufacture of such a medicament.

The compounds of formula I have therapeutic applications in the treatment of diabetes,

Experiments undertaken by the applicant have demonstrated that compounds of formula I are capable of inhibiting glycogen synthase kinase 3 (GSK3). Glycogen synthase kinase 3 is a Ser/Thr protein kinase composed of two isoforms (α and β), which are highly homologous within the catalytic domain. For a recent review on GSK3 biology refer to Frame, S.; Cohen, P. Biochem. J., 2001, 359, 1.

It is known that some CDK inhibitors, including eg. hymenialdisine (Meijer, L.; Thunuissen, A.-M.W.H.; White, AW.; Garnier, M.; Nikolic, M.; Tsai, L.-H.; Waiter, J.; Cleverley, K.E.; Salinas. P.C.; Wu, Y.-Z.; Biernat, J.; Mandelkow, E.M.; Kim, S.-H.; Pettit, G.R Chem. Biol., 2000, 7, 51), paullones (Leost, M.; Schultz, C.; Link A.; Wu, Y.-Z.; Biemat, J.; Mandelkow, B. M; Bibb, J.A.; Snyder, G.L.; Greengaid, P.; Zaharevitz, D.W.; Gussio, R; Senderowicz, A.M.; Sausville. E.A.; Kunick, C.; Meijer, L. Eur. J. Biochem., 2000, 267, 5983), and indirubins (Leclerc, S.; Garnier, M.; Hoessel, R.; Marko, D.; Bibb, J.A..; Snyder, G.L.; Greengard, P.; Biernat, J.; Wu, Y.-Z.; Mandelkow, E.-M.; Eisenbrand, G.; Meijer, L. J. Biol. Chem., 2001, 276,251) also inhibit GSK3. On the other hand, other CDK inhibitor molecules that do not inhibit GSK3, *e.g*. roscovitine (Havlicek, L.; Hanus, J.; Vesely, J.; Leclerc, S.; Meijer, L.; Shaw, G.; Stmad, M. J. Med Chem., 1997, 40, 408) and other purine-based inhibitors (Chang, Y.T.; Gray, N.S.; Rosania, G.R.; Sutherlin, D.P.; Kwon, S.; Norman, T.C.; Sarohia, R.; Leost, M.; Meijer, L.; Schultz, P.G. Chem. Biol., 1999, 6, 361).

One aspect of the invention relates to the use of compounds of formula I, or pharmaceutically accetable salts thereof, in the preparation of a medicament for treating diabetes.

In a particularly preferred embodiment, the diabetes is type II diabetes.

GSK3 is one of several protein kinases that phosphorylate glycogen synthase (GS). The stimulation of glycogen synthesis by insulin in skeletal muscle results from the dephosphorylation and activation of GS. The action of GSK3 on GS thus results in GS deactivation and thus suppression of the conversion of glucose into glycogen in muscles.

Type II diabetes (non-insulin dependent diabetes mellitus) is a multi-factorial disease. Hyperglycaemia is due to insulin resistance in the liver, muscles, and other tissues, coupled with impaired secretion of insulin. Skeletal muscle is the main site for insulin-stimulated glucose uptake, there it is either removed from circulation or converted to glycogen. Muscle glycogen deposition is the main determinant in glucose homeostasis and type II diabetics have defective muscle glycogen storage. There is evidence that an increase in GSK3 activity is important in type II diabetes (Chen, Y.H.; Hansen, L.; Chen, M.X.; Bjorbaek, C.; Vestergaard, H.; Hansen, T.; Cohen, P.T.; Pedersen, O. Diabetes, 1994, 43, 1234). Furthermore, it has been demonstrated that GSK3 is over-expressed in muscle cells of type II diabetics and that an inverse correlation exists between skeletal muscle GSK3 activity and insulin action (Nikoulina, S.E.; Ciaraldi, T.P.; Mudaliar, S.; Mohideen, P.; Carter, L.; Henry, R.R. Diabetes, 2000, 49, 263).

GSK3 inhibition is therefore of therapeutic significance in the treatment of diabetes, particularly type II, and diabetic neuropathy.

A further aspect of the invention relates to a method of treating diabetes comprising inhibiting GSK3 by administering to a subject in need thereof, a therapeutically effective amount of a compound of formula I, or a pharmaceutically acceptable salt thereof, such that treatment of diabetes occurs.

### SALTS/ESTERS

The compounds used in the present invention can be present as salts or esters, in particular pharmaceutically acceptable salts or esters.

Pharmaceutically acceptable salts of the compounds of the invention (first and seconds aspects) include suitable acid addition or base salts thereof A review of suitable pharmaceutical salts may be found in Berge et al, J Pharm Sci, 66 1-19 (1977). Salts are formed, for example with strong inorganic acids such as mineral acids, e.g. sulphuric acid, phosphoric acid or hydrohalic acids; with strong organic carboxylic acids, such as alkanecarboxylic acids of 1 to 4 carbon atoms which are unsubstituted or substituted (e.g., by halogen), such as acetic acid; with saturated or unsaturated dicarboxylic acids, for example oxalic, malonic, succinic, maleic, fumaric, phthalic or tetraphthalic; with hydroxycarboxylic acids, for example ascorbic, glycolic, lactic, malic, tartaric or citric acid; with aminoacids, for example aspartic or glutamic acid; with benzoic acid; or with organic sulfonic acids, such as (C₁-C₄)-alkyl- or aryl-sulfonic acids which are unsubstituted or substituted (for example, by a halogen) such as methane- or p-toluene sulfonic acid.

Esters are formed either using organic acids or alcohols/hydroxides, depending on the functional group being esterified. Organic acids include carboxylic acids, such as alkanecarboxylic acids of 1 to 12 carbon atoms which are unsubstituted or substituted (e.g., by halogen), such as acetic acid; with saturated or unsaturated dicarboxylic acid, for example oxalic, malonic, succinic, maleic, fumaric, phthalic or tetraphthalic; with hydroxycarboxylic acids, for example ascorbic, glycolic, lactic, malic, tartaric or citric acid; with aminoacids, for example aspartic or glutamic acid; with benzoic acid; or with organic sulfonic acids, such as (C₁-C₄)-alkyl- or aryl-sulfonic acids which are unsubstituted or substituted (for example, by a halogen) such as methane- or p-toluene sulfonic acid. Suitable hydroxides include inorganic hydroxides, such as sodium hydroxide, potassium hydroxide, calcium hydroxide, aluminium hydroxide. Alcohols include alkanealcohols of 1-12 carbon atoms which may be unsubstituted or substituted, e.g. by a halogen).

### ENANTIOMERS AND TAUTOMERS

In all aspects of the present invention previously discussed, the invention includes, where appropriate the use of all enantiomers and tautomers of compounds of formula I. The man skilled in the art will recognise compounds that possess an optical properties (one or more chiral carbon atoms) or tautomeric characteristics. The corresponding enantiomers and/or tautomers may be isolated/prepared by methods known in the art.

### POLYMORPHS

The invention furthermore relates to the compounds of use in the present invention in their various crystalline forms, polymorphic forms and (an)hydrous forms. It is well established within the pharmaceutical industry that chemical compounds may be isolated in any of such forms by slightly varying the method of purification and or isolation form the solvents used in the synthetic preparation of such compounds.

### PRODRUGS

The invention further includes the compounds of use in the present invention in prodrug form. Such prodrugs are generally compounds of formula I wherein one or more appropriate groups have been modified such that the modification may be reversed upon administration to a human or mammalian subject. Such reversion is usually performed by an enzyme naturally present in such subject, though it is possible for a second agent to be administered together with such a prodrug in order to perform the reversion in vivo. Examples of such modifications include ester (for example, any of those described above), wherein the reversion may be carried out be an esterase etc. Other such systems will be well known to those skilled in the art.

### PHARMACEUTICAL COMPOSITIONS

The present invention also encompasses the use of pharmaceutical compositions comprising the compounds of the invention. In this regard, and in particular for human therapy, even though the compounds of the present invention (including their pharmaceutically acceptable salts, esters and pharmaceutically acceptable solvates) can be administered alone, they will generally be administered in admixture with a pharmaceutical carrier, excipient or diluent selected with regard to the intended route of administration and standard pharmaceutical practice.

Thus, the present invention also relates to the use of pharmaceutical compositions comprising one or more compounds of formula I or pharmaceutically acceptable salts or esters thereof, together with at least one pharmaceutically acceptable excipient, diluent or carrier.

By way of example, in the pharmaceutical compositions of the present invention, the compounds of use in the invention may be admixed with any suitable binder(s), lubricant(s), suspending agent(s), coating agent(s), and/or solubilising agent(s). Examples of such suitable excipients for the various different forms of pharmaceutical compositions described herein may be found in the "Handbook of Pharmaceutical Excipients, 2nd Edition, (1994), Edited by A Wade and PJ Weller.

### ADMINISTRATION

The pharmaceutical compositions of the present invention may be adapted for oral, rectal, vaginal, parenteral, intramuscular, intraperitoneal, intraarterial, intrathecal, intrabronchial, subcutaneous, intradermal, intravenous, nasal, buccal or sublingual routes of administration.

For oral administration, particular use is made of compressed tablets, pills, tablets, gellules, drops, and capsules. Preferably, these compositions contain from 1 to 250 mg and more preferably from 10-100 mg, of active ingredient per dose.

Other forms of administration comprise solutions or emulsions which may be injected intravenously, intraarterially, intrathecally, subcutaneously, intradermally, intraperitoneally or intramuscularly, and which are prepared from sterile or sterilisable solutions. The pharmaceutical compositions of the present invention may also be in form of suppositories, pessaries, suspensions, emulsions, lotions, ointments, creams, gels, sprays, solutions or dusting powders.

An alternative means of transdermal administration is by use of a skin patch. For example, the active ingredient can be incorporated into a cream consisting of an aqueous emulsion of polyethylene glycols or liquid paraffin. The active ingredient can also be incorporated, at a concentration of between 1 and 10% by weight, into an ointment consisting of a white wax or white soft paraffin base together with such stabilisers and preservatives as may be required.

Injectable forms may contain between 10 - 1000 mg, preferably between 10 - 250 mg, of active ingredient per dose.

Compositions may be formulated in unit dosage form, i.e., in the form of discrete portions containing a unit dose, or a multiple or sub-unit of a unit dose.

### DOSAGES

A person of ordinary skill in the art can easily determine an appropriate dose of one of the instant compositions to administer to a subject without undue experimentation. Typically, a physician will determine the actual dosage which will be most suitable for an individual patient and it will vary with the age, weight and response of the particular patient. The dosages disclosed herein are exemplary of the average case. There can of course be individual instances where higher or lower dosage ranges are merited, and such are within the scope of this invention.

In an exemplary embodiment, one or more doses of 10 to 150 mg/day will be administered to the patient for the treatment of a viral disorder.

Preferably, said compound of formula I is administered in an amount sufficient to inhibit GSK3.

Even more preferably, said compound of formula I is administered in an amount sufficient to inhibit GSK3β.

### COMBINATIONS

In a particularly preferred embodiment, the one or more compounds of the invention are administered in combination with one or more other pharmacologically active agents. In such cases, the compounds of the invention may be administered consecutively, simultaneously or sequentially with the one or more other pharmacologically active agents.

It is known in the art that many drugs are more effective when used in combination. In particular, combination therapy is desirable in order to avoid an overlap of major toxicities, mechanism of action and resistance mechanism(s). Furthermore, it is also desirable to administer most drugs at their maximum tolerated doses with minimum time intervals between such doses. The major advantages of combining drugs are that it may promote additive or possible synergistic effects through biochemical interactions and also may decrease the emergence of drug resistance which would have been otherwise responsive to initial treatment with a single agent.

Beneficial combinations may be suggested by studying the pharmacological activity of the test compounds with agents known or suspected of being valuable in the treatment of a particular disorder. This procedure can also be used to determine the order of administration of the agents, i.e. before, simultaneously, or after delivery.

The present invention is now described by way of example, and with reference to the accompanying figures, wherein:
Figure 1 shows the activation of cellular glycogen synthase activity by example compounds [20] and [21] in HEK293 cells (top), 3T3 mouse adipocyte cells (middle) and L6 rat myocyte cells (bottom), as measured by the fractional reaction velocity of the enzyme (the ratio between the activity at 0.1 and 10 mM glucose-6 phosphate substrate concentrations).
Figure 2 shows blood glucose levels (mmol/L) against time (minutes) for compound [20] in ZDF rats.

### EXAMPLES

Table 1 shows the structures and chemical names of the exemplified compounds.

### Example 1

### 3,5-Dimethyl-1H-pyrrole-2-carbonitrile

Ethyl cyanoacetate (10 mL, 94 mmol) was diluted with AcOH (20 mL) and the solution was cooled to -10°C (ice-MeOH bath). NaNO₂ (6.5 g, 94 mmol) was dissolved in H₂O (10 mL) and the solution was added dropwise over a period of 40 min, keeping the internal temperature < 0° C. After completion of the addition, the reaction mixture was stirred for 1 h with cooling. It was then warmed to room temperature and stirred for a further 3 h. The mixture was diluted with AcOH (50 mL) and H₂O (50 mL). Pentane-2,4-dione (10.6 mL, 103 mmol) was added and the mixture heated to~75°C. To this reaction mixture Zn powder (6.9 g, 105 mmol) was added in portions over a period of 30 min at such a rate as to maintain the internal temperature < 90°C. The reaction mixture was then heated for a further 30 min before pouring into H₂O (1 L). From the reaction mixture title compound (3.67 g) was filtered as an off-white solid. The filtrate was extracted with EtOAc (3 x 500 mL). The combined organic extracts were washed (brine) and dried (MgSO₄). The solvent was evaporated to a brown oil, which was purified by chromatography (100 g SiO_{2'} eluted with 4:1 heptane / EtOAc) to afford a further crop (4.41 g) of this product as a pale yellow solid (total yield 72 %).

### 4-Acetyl-3,5-dimethyl-1H pyrrole-2-carbonitrile

3,5-Dimethyl-1H pyrrole-2-carbonitrile (1.2 g, 10 mmol) was dissolved in anhydrous 1,2-dichloroethane (15 mL) and AlCl₃ (2.93 g, 22 mmol) was added in portions. The reaction vessel was purged with N₂ and was cooled in an ice-water bath. AcCl (0.71 mL, 10 mmol) was added dropwise and the mixture was stirred for 1 h with cooling and for a further 3 h at room temperature. The reaction mixture was quenched by careful addition of 2 M aq HCl. The acidity of the mixture was adjusted to approximately pH 6 by addition of NaHCO₃. After separation of the organic phase, the aqueous phase was extracted with EtOAc (3 x 100mL). The combined organic phases were washed (H₂O, then brine), dried (MgSO₄), and filtered. The solvent was evaporated to afford the title compound (1.42 g, 88 %) as a pale tan solid. ¹H-NMR (CDCl₃) δ: 2.44 (s, 3H, CH₃), 2.45 (s, 3H, CH₃), 2.54 (s, 3H, CH₃), 8.75 (br. s, 1H, NH).

### 4-(3 Dimethylamino-acryloyl)-3,5-dimethyl-1H-pyrrole-2-carbonitrile

4-Acetyl-3,5-dimethyl-1*H* pyrrole-2-carbonitrile (1.38 g, 8.51 mmol) was suspended in 1,1-bis-dimethylamino-3,3-dimethyl-butan-2-one (1.3 mL) and heated at 75°C for 42 h. The reaction mixture was evaporated to dryness and the residue was purified by SiO₂ chromatography (heptane / EtOAc) to afford the title compound (1.2 g, 65 %) as a pale tan solid.¹H-NMR (DMSO-d₆) δ: 2.21 (s, 3H, CH₃), 2.31 (s, 3H, CH₃), 3.32 (s, 6H, CH₃), 5.22 (d, 1H, *J*=12.4 Hz, CH), 7.47 (d 1H, *J*= 12.4 Hz, CH), 11.96 (br. s, 1H, NH).

### 3,5-Dimethyl-4-[2-(3-nitro-phenylamino)-pyrmidin-4-yl]-1H-pyrrole-2-carbonitrile [1]

To a mixture of 4-(3-dimethylamino-acryloyl)-3,5-dimethyl-1*H*-pyrrole-2-carbonitrile (1.0 mmol, 0.22 g) and 3-nitrophenyl guanidine nitrate (1.5 mmol, 0.36 g) in 2-methoxyethanol (5 mL) was added K₂CO₃ (138 mg, 1.0 mmol). The reaction mixture was heated at 120 °C under N₂ for 18 h. The solvent was evaporated to dryness and the residue was purified by flash chromatography (1:2 EtOAc / heptane) to afford the title compound as a light-yellow solid. M.p. 258-259 °C. MS: [M+H]⁺ = 336.1 (C₁₇H₁₄N₆O₂ requires 334.3). ¹H-NMR (CD₃OD) *δ:* 2.39 (s, 3H, CH₃), 2.49 (s, 3H, CH₃), 6.94 (d, 1H, *J*= 5.1 Hz, pyrimidinyl-H), 7.50 (t, 1H, *J =* 8.3 Hz, Ph-H), 7.81 (m, 1H, Ph-H), 7.94 (m, 1H, Ph-H), 8.45 (d, 1H, *J*= 5.1 Hz, pyrimidinyl-H), 8.94 (t, 1H, *J*= 2.2 Hz, Ph-H).

The following compounds were prepared in a manner analogous to that described above:

### 4-[2-(4-Fluoro-phenylamino)-pyrimidin-4-yl]-3, 5-dimethyl-1H-pyrrole-2-carbonitrile [2]

MS: [M+H]⁺ = 307.7 (C₁₇H₁₄FN₅ requires 307.3). ¹H-NMR (DMSO-d₆) δ: 2.30 (s, 3H, CH₃), 2.40 (s, 3H, CH₃), 6.84 (d, 1H, *J* = 5.0 Hz, pyrimidinyl -H), 7.00 (m, 2H, Ph-H), 7.73 (m, 2H, Ph-H), 8.40 (d, 1H, *J*=5.5Hz, pyrimidinyl -H), 9.46 (s, 1H, NH), 12.19 (br. s, 1H.NH).

### 4-[2-(4-Hydroxy-phenylamino)-pyrimidin-4-yl]-3,5-dimethyl-1H-pyrrole-2-carbonitrile [3]

M.p. 272-276 °C. MS: [M+H]⁺ =305.8 (C₁₇H₁₅N₅O requires 305.3).¹H-NMR (CD₃OD) δ: 2.33 (s, 3H, CH₃), 2.41 (s, 3H, CH₃), 6.74-6.56 (m, 3H, pyrimidinyl-H/Ph-H), 7.36 (d, 2H, J= 8.5 Hz, Ph-H), 8.25 (d, 1H, *J=* 5.4 Hz, pyrimidinyl-H).

### 3,5-Dimethyl-4-[2-(4-trifluoromethyl-phenylamino)-pyrimidin-4-yl]-1H-pyrrole-2-carbonitrile [4]

M.p. 195.6-198.9 °C. MS: [M+H]⁺ = 357.7 (C₁₈H₁₄F₃N₅ requires 357.3). ¹H-NMR (CDCl₃)δ: 2.33 (s, 3H, CH₃), 2.44 (s, 3H, CH₃), 6.75 (d, 1H, *J*= 5.1 Hz, pyrimidinyl-H, 7.20 (br. s, 1H, NH), 7.50 (d, 2H, J= 8.8 Hz, Ph-H), 7.71 (d, 2H, *J*= 8:8 Hz, Ph-H), 8.39 (d, 1H, *J*= 5.1 Hz, pyrimidinyl), 8.40 (br. s, 1H, NH).

### 4-[2-(4-Iodo-phenylamino)-pyrimidin-4-yl]-3,5-dimethyl-1H-pyrrole-2-carbonitrile [5]

M.p. 178.3-181.2 °C. MS: [M+H]⁺ = 416.6 (C₁₈H₁₄IN₅ requires 415.2). ¹H-NMR (CDCl₃) δ: 2.39 (s, 3H, CH₃), 2.49 (s, 3H, CH₃), 6.76 (d, 1H, *J* = 5.1 Hz, pyrimidinyl-H), 7.10 (br. s, 1H, NH), 7.44 (d, 2H, *J*= 8.8 Hz, Ph-H), 7.61 (d, 2H, *J*= 8.8 Hz, Ph-H), 8.42 (d, 1H, *J*= 5.1 Hz, pyrimidinyl-H), 8.45 (br. s, 1H, NH).

### 4-[2-(3-Hydroxy-phenylamino)-pyrimidin-4-yl]-3,5-dimethyl-1H-pyrrole-2-carbonitrile [6]

M.p. 247-250 °C. MS: [M+H]⁺ =305.8 (C₁₇H₁₅N₅O requires 305.3). ¹H-NMR (DMSO-d₆) *δ:* 2.31 (s, 3H, CH₃), 2.41 (s, 3H, CH₃), 6.33 (m, 1H, Ph-H), 6.82 (d, 1H, *J* = 5.1 Hz, pyrimidinyl-H), 7.01 (t, 1H, *J* = 8.1 Hz, Ph-H), 7.11 (m, 1H, Ph-H), 7.33 (t, 1H, *J* = 2.1 Hz, Ph-H), 8.40 (d, 1H, *J*= 5.4 Hz, pyrimidinyl-H), 9.18 (s, 1H), 9.30 (s, 1H), 12.20 (br. s, 1H, NH).

### 3,5 Dimethyl-4-[2-(4-methyl-3-nitrophenylamino) pyrimidin-4 yl]-1H pyrrole-2-carbonitrile [7]

M.p. 233-237 °C. MS: [M+H]⁺ = 350.0 (C₁₈H₁₆N₆O₂ requires 348.6). ¹H-NMR (DMSO-d₆) *δ:* 2.31 (s, 3H, CH₃), 2.42 (s, 3H, CH₃), 2.44 (s, 3H, CH₃), 6.92 (d, 1H, *J* = 5.4 Hz, pyrimidinyl-H), 7.39 (d, 1H, *J*= 8.5 Hz, Ph-H), 7.87 (dd, 1H, *J*= 8.1, 1.7 Hz, Ph-H), 8.48 (d, 1H, *J* = 5.1 Hz, pyrimidinyl-H), 8.63 (d, 1H, *J* =1.7 Hz, Ph-H), 9.87 (s, 1H, NH), 12.21 (br. s, 1H, NH).

### 4-[2-(3 Iodo-4-methylphenylamino) pyrimidin-4 yl]-3,5-dimethyl-1H- pyrrole-2-carbonitrile [8]

M.p. 189.5-191.7 °C. MS: [M+H]⁺ = 431.5 (C₁₈H₁₆IN₅ requires.429.6). ¹H-NMR (DMSO-d₆) δ: 2.29 (s, 3H, CH₃), 2.32 (s, 3H, CH₃), 2.43 (s, 3H, CH₃), 6.85 (d, 1H, *J*= 5.1 Hz, pyrimidinyl-H), 7.20 (d, 1H, *J* = 8.1 Hz, Ph-H), 7.57 (m, 1H, Ph-H), 8.41-8.43 (m, 2H, Ph-H, pyrimidinyl-H), 9.48 (s, 1H, NH), 12.20-(br. s; 1H, NH).

### 4-[2-(4-Chloro-3-methyl-phenylamino)-pyrimidin-4-yl]-3,5-dimethyl-1H-pyrrole-2-carbonitrile [9]

M.p. 194.2-197.9 °C. MS: [M+H]⁺= 338.0 (C₁₈H₁₆ClN₅ requires 337.8). ¹H-NMR (DMSO-d₆) δ: 2.27 (s, 3H, CH₃), 2.31 (s, 3H, CH₃), 2.41 (s, 3H, CH₃), 6.86 (d, 1H, J= 5.4 Hz, pyrimidinyl-H), 7.2 8 (d, 1H, *J* = 8.5 Hz, Ph-H), 7.61 (dd, 1H, *J* = 8.8, 2.4 Hz, Ph-H), 7.73 (d, 1H, *J*= 2.7 Hz, Ph-H), 8.43 (d, 1H, *J*= 5.1 Hz, pyrimidinyl-H), 9.51 (s, 1H, NH), 12.21 (br. s, 1H, NH).

### 4-[2-(3-Hydroxy-4-methyl-pheizylamino)-pyrimidin-4-yl]-3,5-dimethyl-1H-pyrrole-2-carbonitrile[10]

M.p. 221-225 °C. MS: [M+H]⁺ = 320.9 (C₁₈H₁₇N₅O requires 319.4). ¹H-NMR (DMSO-d₆) δ: 2.03 (s, 3H, CH₃), 2.29 (s, 3H, CH₃), 2.40 (s, 3H, CH₃), 6.78 (d, 1H, *J* = 5.1 Hz, pyrimidinyl-H), 6.89 (d, 1H, *J*= 8.1 Hz, Ph-H), 7.02 (dd, 1H, *J*= 8.3, 1.7Hz, Ph-H), 7.29 (d, 1H, *J* = 0.7 Hz, Ph-H), 8.37 (d, 1H, J= 4.9 Hz, pyrimidinyl-H), 9.08 (s, 1H), 9.20 (s, 1H), 12.17 (br. s, 1H, NH).

### 4-[2-(4-Fluoro-3-methyl-phenylamino)-pirimidin-4-yl]-3,5-dimethyl-1H-pyrrole-2-carbonitrile [11]

M.p. 161.3-164.1 °C. MS: [M+H]⁺ = 321.6 (C₁₈H₁₆FN₅ requires 321.4). ¹H-NMR. (DMSO-d₆) δ: 2.19 (s, 3H, CH₃), 2.30 (s, 3H, CH₃), 2.42 (s, 3H, CH₃), 6.82 (d, 1H, *J* = 5.1 Hz, pyrimidinyl-H), 7.03 (t, 1H, *J* = 9.3 Hz, Ph-H), 7.53 (m, 1H, Ph-H), 7.61 (dd, 1H, *J* = 7.1, 2.4 Hz, Ph-H), 8.39 (d, 1H, J= 5.1 Hz, pyrimidinyl-H), 9.36 (s, 1H, NH), 12.20 (br. s, 1H, NH).

### 4-[2-(4-Dimethylamino-phenylamino)-pyrimidin-4-yl]-3,5-dimethyl-1H-pyrrole-2-carbonitrile [12]

M.p. 190.6-193.7 °C. MS: [M+H]⁺ = 334.7 (C₁₉H₂₀N₆ requires 332.4). ¹H-NMR (CDCl₃) 8: 2.36 (s, 3H, CH₃), 2.46 (s, 3H, CH₃), 2.94 (br. s, 6H, CH₃), 6.66 (d, 1H, *J* = 5.6 Hz, pyrimidinyl-H), 6.79-6.80 (m, 2H, Ph-H), 7.05 (br. s, 1H, NH), 7.40-7.43 (m, 2H, Ph-H), 8.34 (d, 1H, *J* = 5.1Hz, pyrimidinyl-H), 8.52 (br.s, 1H, NH).

### Example 2

### 4-(3 Dimethylamino-acryloyl)-3,5-dimethyl-1H-pyrrole-2-carboxylic acid amide.

1-(2,4-Dimethyl-1*H* pyrrol-3-yl)-ethanone (1.1 g, 10 mmol) was partially dissolved in a 2 M solution of NH₃ in MeOH and H₂O₂ (10 mL of a 27 % w/w solution in H₂O) was added dropwise over a period of 40 min at such a rate as to maintain the internal temperature ≤ 30 °C. The mixture was stirred for 18 h at room temperature. The resulting suspended white solid was filtered and recrystallised from EtOAc to afford 4-acetyl-3,5-dimethyl-1H-pyrrole-2-carboxylic acid amide (1.06 g). An aliquot (720 mg, 4 mmol) was suspended in 1,1-bis-dimethylamino-3,3-dimethyl-butan-2-one (2 mL, 9.6mmol) in a N₂-flushed flask and heated at 75°C for 48 h. The crude mixture was cooled and purified by SiO₂ chromatography (EtOAc / MeOH gradient elution). The title compound (449 mg) was obtained as a buff solid. ¹H-NMR (DMSO-d₆) δ: 2.30 (s, 3H, CH₃), 2.46 (s, 3H, CH₃), 2.90 (br. s, 2H, NH). 3.09 (s, 3H, CH₃), 3.13 (s, 3H, CH₃), 5.23 (d, 1H, *J* = 12.4 Hz, CH), 7.38 (d, 1H, *J* =12.7 Hz, CH), 10.97 (br. s, 1H, NH).

### 4-[2-(4-Fluoro-phenylamino)-pyrimidin-4-yl]-3,5-dimethyl-1H-pyrrole-2-carboxylic acid amide [13]

4-(3-Dimethylamino-acryloyl)-3,5-dimethyl-1*H*-pyrrole-2-carboxylic acid amide (100 mg, 0.43 mmol), 4-fluorophenylguanidine nitrate (139 mg, 0.65 mmol) and K₂CO₃ (94 mg, 0.68 mmol) were partially dissolved in 2-methoxyethanol (5 mL) and heated at 120°C for 18 h. The mixture was concentrated *in vacuo* and purified by SiO₂ chromatography (EtOAc / MeOH gradient elution). The crude product was triturated in iPr₂O to afford the title compound (31 mg) as a buff solid. M.p. 93.5-96.8 °C. MS: [M+H⁺] = 326.9 (C₁₇H₁₆FN₅O requires 325.3). ¹H-NMR (DMSO-d₆) δ: 2.36 (s, 3H, CH₃), 2.39 (s, 3H, CH₃), 6.79 (d, 1H, *J* = 5.4 Hz, pyrimidinyl-H), 6.92 (br. s, 2H, NH), 7.07 (t, 2H, *J* = 8.5 Hz, Ph-H), 7.76-7.78 (m, 2H, Ph-H), 8.36 (d, 1H, J= 5.4 Hz, pyrimidinyl-H) 9.41 (s, 1H, NH),11.24 (br. s, 1H, NH).

### Example 3

### 3-Dimethylamino-1-(2,4-dimethyl-5-nitro-1H-pyrrol-3-yl)-propenone

HNO₃ (0.28 mL of a 69 % w/v aq solution, 4.37 mmol) was added dropwise to Ac₂O (5 mL) at room temperature, keeping the internal temperature ≤ 25 °C. The nitrating mixture was stirred at room temperature for 15 min before cooling to -40 °C. 1-(2,4-Dimethyl-1*H-*pyrrol-3-yl)-ethanone (500 mg, 3.64 mmol) was dissolved in Ac₂O (6 mL) and added dropwise, keeping the internal temperature s -30 °C. The mixture was stirred at -40 °C for 30 min then at -10 °C for a further 30 min. The mixture was poured into ice-water (50 mL) and was extracted with Et₂O (3 × 60 mL). The combined organic extracts were washed (brine), dried (Na₂SO₄), filtered, and evaporated *in vacuo* to give a dark brown solid. This was recrystallised from MeOH to afford 1-(2,4-dimethyl-5-nitro-1*H*-pyrrol-3-yl)-ethanone (158 mg). An aliquot (150 mg, 0.82 mmol) was suspended in 1,1-bis-dimethylamino-3,3-dimethyl-butan-2-one (0.42 mL, 2.02 mmol) in a N₂-flushed flask and was heated at 70 °C for 18 h. The mixture was triturated in EtOAc to afford the title compound (119 mg) as a brown solid. ¹H-NMR (DMSO-d₆) δ: 2.30 (s, 3H, CH₃), 2.40 (s, 3H, CH₃), 2.80 (br. s, 3H, CH₃), 3.07 (br. s, 3H, CH₃), 5.19 (d, 1H, *J=*12.7 Hz, CH), 7.45 (d, 1H, *J=12.4* Hz, CH), 12.76 (br, 1H, NH).

### [4-(2,4-Dimethyl-5-nitro-1H-pyrrol-3-yl)-pyrimidin-2-yl]-(4-fluoro-phenyl)-amine [14]

*3-Dimethylamino-1-(2,4-dimethyl-5-nitro-1H-pyrrol-3-yl)-propenone* (110 mg, 0.46 mmol), 4-fluorophenyl guanidine nitrate (150 mg, 0.7 mmol), and K₂CO₃ (193 mg, 1.4 mmol) were partially dissolved in 2-methoxyethanol and heated at 120 °C for 18 h. The mixture was concentrated *in vacuo* and purified by SiO₂ chromatography (heptane *l* EtOAc gradient elution). The crude product was triturated in iPr₂O to afford the title compound (22 mg) as a pale orange solid. M.p. 166.3-170.1 °C. MS: [M+H]⁺ = 329.3 (C₁₆H₁₄FN₅O₂ requires 327.3). ¹H-NMR (DMSO-d₆) δ: 2.49 (s, 3H, CH₃), 2.59 (s, 3H, CH₃), 6.73 (d, 1H, *J*= 5.1 Hz, pyrimidinyl-H), 7.04 (t, 2H, *J=* 8.8 Hz, Ph-H), 7.07 (br. s, 1H, NH), 7.55-7.58 (m, 2H, Ph-H), 8.44 (d, 1H, *J=* 5.1 Hz, pyrimidinyl-H), 9.40 (br. s, 1H, NH).

The following compound was prepared in analogous manner:

### N-[4-(2,4-Diniethyl-5-nitro-1H-pyrrol-3-yl)-pyrimidin-2-yl]-N,N'-dimethyl-benzene-1,4-diamine [15]

M.p. 265-268 °C. MS: [M+H]⁺ = 353.0 (C₁₈H₂₀N₆O₂ requires 352.4). ¹H-NMR (DMSO-d₆) δ*:* 2.39 (s, 3H, CH₃), 2.48 (br. s, 6H, CH₃), 2.82 (s, 3H, CH₃), 6.69 (d, 2H, *J=* 9.0 Hz, Ph-H), 6.74 (d, 1H, *J=* 5.1 Hz, pyrimidinyl-H), 7.50 (d, 2H, *J=* 9.0 Hz, Ph-H), 8.38 (d, 1H, *J* = 5.1 Hz, pyrimidinyl-H), 9.18 (s, 1H, NH), 13.00 (br. s, 1H, NH).

### Example 4

### [4-(5-Bromo-2,4-dimethyl-1H-pyrrol-3-yl)-pyrimidin-2-yl]-(4-fluoro-phenyl)-amine [16]

[4-(2,4-Dimethyl-1*H*-pyrrol-3-yl)-pyrimidin-2-yl]-(4-fluoro-phenyl)-amine (80 mg, 0.28 mmol) was dissolved in THF (4 mL) and cooled to -50 °C. N Bromosuccinimide (55 mg, 0.31 mmol) was dissolved in THF (2 mL) and added dropwise, keeping the internal temperature ≤ -40 °C. The mixture was stirred for 1h with cooling then evaporated *in vacuo.* The residue was treated with H₂O (10 mL) and extracted with EtOAc (3 x 10mL). The combined organic extracts were washed (brine), dried (MgSO₄), filtered, and evaporated *in vacuo.* The crude product was purified by SiO₂ chromatography heptane / EtOAc gradient elution) to afford the title compound (19 mg) as an orange solid after recrystallisation from iPr₂O. M.p. 181.4-183.3 °C. MS: [M+H]⁺ = 362.9 (C₁₆H₁₄BrFN₄ requires 361.2).¹H-NMR (CDCl₃) δ: 2.10 (s, 3H, CH₃), 2.36 (s, 3H, CH₃) 6.56 (d, 1H, *J*= 5.1 Hz, pyrimidinyl-H), 6.97 (t, 2H, *J*= 8.3Hz, Ph-H), 7.00 (br. s, 1H, NH), 7.79-7.52 (m, 2H, Ph-H), 8.85 (br. s, 1H, NH), 8.26 (d, 1H, *J*= 5.1 Hz, pyrimidinyl-H).

The following compound was prepared in analogous manner:

### [4-(5-Bromo-2,4-dimethyl-1H-pyrrol-3-yl)-pyrimidin-2-yl]-(3-nitro-phenyl)-amine [17]

M.p. 198.1-203 °C. MS: [M+H]⁺ =389.3 (C₁₆H₁₄BrN₅O₄ requires 361.2). ¹H-NMR (CDCl₃) δ: 2.49 (s, 3H, CH₃), 2.59 (s, 3H, CH₃) 6.73 (d, 1H, *J*= 5.1 Hz, pyrimidinyl-H), 7.04 (t, 2H, *J*= 8.8 Hz, Ph-H), 7.57 (m, 2H, Ph-H), 7.90 (br. s, 1H, NH), 8.44 (d, 1H, *J=* 5.1 Hz, pyrimidinyl-H), 9.40 (br. s, 1H, NH).

### Example 5

### [4-(5-Chloro-2,4-dimethyl-1H-pyrrol-3-yl)-pyrimidin-2-yl]-(4-fluoro-phenyl)-amine [18]

[4-(2,4-Dimethyl-1*H*-pyrrol-3-yl)-pyrimidin-2-yl]-(4-fluoro-phenyl)-amine (80 mg, 0.28 mmol) was dissolved in THF (4 mL) and cooled to -60 °C. *N*-Chlorosuccinimide (41 mg, 0.31 mmol) was dissolved in THF (2 mL) and added dropwise, keeping the internal temperature ≤ -50 °C. The mixture was stirred for 30min with cooling then evaporated *in vacuo.* The residue was treated with H₂O (10 mL) and extracted with EtOAc (3 x 10 mL). The combined organic extracts were washed (brine), dried (MgSO₄), filtered, and evaporated *in vacuo.* The crude product was purified by SiO₂ chromatography (heptane / EtOAc gradient elution) to afford the title compound (37 mg) as an orange solid after recrystallisation from iPr₂O. M.p. 200-203 °C. MS: [M+H]⁺ = 317.7 (C₁₆H₁₄CIFN₄ requires 316.8). ¹H-NMR (CDCl₃) δ:2.17 (s, 3H, CH₃), 2.42 (s, 3H, CH₃) 6.77 (d, 1H, *J =* 5.9 Hz, pyrinudinyl-H), 7.02-7.06 (m, 3H, Ph-h, NH), 7.54-7.56 (m, 2H, Ph-H), 7.95 (br. s, 1H, NH), 8.25 (d, 1H, J= 5.4 Hz, pyrimidinyl-H).

### Example 6

### [4-(5-Dimethylaminomethyl-2,4-dimethyl-1H-pyrrol-3-yl)-pyiimidin-2-yl]-(4-fluorophenyl)-amine [19]

Dimethylamine (40 µL, 0.31 mmol) was diluted with methanol (0.5 mL) and formaldehyde (30 µL of a 37 % w/w aq solution, 0.37 mmol) was added. [4-(2,4-Dimethyl-1H-pyrrol-3-yl)-pyrimidin-2-yl]-(4-fluoro-phenyl)-amine (87 mg, 0.31 mmol) was added in small portions and the mixture was heated to reflux. After 1.5 h the mixture was diluted with H₂O (10 mL). The resulting precipitate was filtered and triturated in 2 M aq HCl. The mixture was filtered and the filtrate was washed with 2 M aq NaOH. The filtrate was extracted with EtOAc (3 × 15 mL). The combined organic extracts were washed (brine), dried (MgSO₄), filtered, and evaporated *in vacuo.* The crude product was purified by SiO₂ chromatography (heptane / EtOAc gradient elution) to afford the title compound (36 mg) as an orange solid after recrystallisation from iPr₂O. M.p. 88.4-91.6 °C. MS: [M+H]⁺= 340.6 (C₁₉H₂₂FN₅ requires 339.4).¹H-NMR (CDCl₃) δ: 2.18 (s, 3H, CH₃), 2.56 (s, 6H, CH₃), 2.42 (s, 3H, CH₃), 3.38 (s, 2H, CH₂), 6.75 (d, 1H, *J=* 5.4 Hz, pyrimidinyl-H), 7.00 (t, 2H, *J*= 8.6 Hz, Ph-H), 7.13 (br. s, 1H, NH), 7.56-7.59 (m, 2H, Ph-H), 8.31 (d, 1H, *J=* 5.1 Hz, pyrimidinyl-H), 8.55 (br. s, 1H, NH).

### Example 7

### 4-[2-(4-Dimethylwnino-3-nitro-phenylamino)-pyrimidin-4-yl]-3,5-dimethyl-1H-pyrrole-2-carbonitrile [20]

This was obtained by reaction of 4-(3-dimethylamino-acryloyl)-3,5-dimethyl-1*H*- pyrrole-2-carbonitrile and N (4-dimethylamino-3-nitro-phenyl)-guanidine nitrate. The crude product was recrystallised from PhMe (21 %) as a yellow solid. ¹H NMR (CD₃OD) δ: 2.38 (s, 3H, CH₃), 2.51 (s, 3H, CH₃), 2.86 (s, 6H, CH₃), 6.76 (d, 1H, *J =* 5.1 Hz, pyrimidinyl-H), 7.02 (s, 1H, Ph-H), 7.04 (m, 1H, Ph-H), 7.49 (m, 1H, Ph-H), 8.29 (d, 1H, *J* = 2.7Hz, Ph-H), 8.40 (d, 1H, *J* = 5.4 Hz, pyrimidinyl-H), 8.94 (t, 1H, *J* = 2.2 Hz, Ph-H). MS *m*/*z* 378.71 (M+H)⁺ (C₁₉H₁₉N₇O₂ requires 377.40).

### Example 8

### N⁴-[4-(2,4-Dimethyl-5-nitro-1H-pyrrol-3-yl)-pyrimidin-2-yl]-N¹,N¹-dimethyl-2-nitrobenzene-1,4-diamine [21]

This was prepared by reaction of 3-dimethylamino-1-(2,4-dimethyl-5-nitro-1*H*-pyrrol-3-yl)-propenone and *N*-(4-dimethylamino-3-nitro-phenyl)-guanidine nitrate. ¹H-NMR (CDCl₃) δ: 2.52 (s, 3H, CH₃), 2.59 (s, 3H, CH₃), 2.87 (s, 6H, CH₃), 6.76 (d, 1H, *J* = 5.1 Hz, pyrimidinyl-H), 7.05 (m, 2H, Ph-H & NH), 7.48 (m, 1H, Ph-H), 8.32 (br. s, 1H, Ph-H), 8.44 (d, 1H, *J* = 5.1 Hz, pyrimidinyl-H).

### Example 9

### 4-[2-(3-Fluoro-4-methyl-phenylamino) pyrimidin-4 yl]-3,5-dimethyl-1H-pyrrole-2-carbonitrile [22]

This compounds was prepared according to the general methods described in Example 1. M.p. 177.7-179.9 °C. MS: [M+H]⁺ = 322.5 (C₁₈H₁₆FN₅ requires 321.3). ¹H-NMR (DMSO-d₆) δ: 2.15 (s, 3H, CH₃), 2.30 (s, 3H, CH₃), 2.43 (s, 3H, CH₃), 6.86 (d, 1H, *J* = 5.1 Hz, pyrimidinyl-H), 7.13 (t, 1H, *J=* 9.0 Hz, Ph-H), 7.36 (dd, 1H, *J=* 8.1, 1.7 Hz, Ph-H), 7.75 (dd, 1H, *J*= 12.9, 1.5 Fiz, Ph-H), 8.43 (d, 1H, *J* = 5.4Hz, pyrimidinyl-H), 9.56 (s, 1H, NH), 12.21 (br. s, 1H, NH).

### Example 10

### 5-[2-(4-Hydroxy-phenylamino)-pyrimidin-4-yl]-3,4-dimethyl-3H-thiazol-2-one [34]

To an ice-cooled solution of potassium thiocyanate (5.67 g, 58 mmol) in Me-2CO (45 mL) was added 3-chloro-pentane-2,4-dione (6.95 mL, 58 mmol) dropwise. After completion of the addition the reaction mixture was warmed to room temperature and stirred for a further 6 h. The solvent was evaporated to dryness. The residue was dissolved in EtOH (30 mL) and HCl (conc. aq, 15 mL) was added. The mixture was heated to reflux for 14 h. It was concentrated and the precipitate was collected, washed with cold MeOH and then Et₂O to afford 9.1 g of a pale solid. This compound was treated with *N,N-* dimethylformamide dimethylacetal (13 mL) at 100 -110 °C for 8 h. The reaction mixture was concentrated and the residue was purified by SiO₂ flash chromatography (EtOAc/PE) to afford 5-(3-dimethylamino-acryloyl)-3,4-dimethyl-3H-thiazol-2-one. ¹H-NMR (CDCl₃) δ: 2.50 (s, 3H, CH₃), 3.07 (s, 3H, CH₃), 3.21 (s, 6H, CH₃), 5.09 (d, 1H, *J*= 12.0 Hz, CH), 7.59 (d, 1H, *J=* 12.0 Hz, CH).

A solution of 5-(3-dimethylamino-acryloyl)-3,4-dimethyl-3*H*-thiazol-2-one (0.23 g, 1.0 mmol) in 2-methoxylethanol (3 mL) was treated with *N*-(4-hydroxy-phenyl)-guanidine nitrate (0.42 g, 2.0 mmol). After refluxing for 20 h the reaction mixture was concentrated and purified by SiO₂ flash chromatography (EtOAc). Recrystallisation from EtOAc afforded the tilted compound (25 mg) as brown crystals. Anal. RP-HPLC: t_{R} = 11.8 min (0 - 60 % MeCN in 0.1 % aq CF₃COOH over 20 min, 1 mL/mi.n, purity > 95%). ¹H-NMR (DMSO-d₆) δ: 2.52 (s, 3H, CH₃), 3.27 (s, 3H, CH₃), 6.68 (d, 2H, *J=* 8.9 Hz, Ph-H), 6.81 (d, 1H, *J=* 5.5 Hz, pyrimidinyl-H), 7.44 (d, 2H, *J=* 8.9 Hz, Ph-H), 8.34 (d, 1H, *J=* 5.5 Hz, pyrimidinyl-H), 9.12 (br. s, 1H, OH/NH), 9.24 (br. s, 1H, NH/OH).

The following compounds were prepared in a similar manner to the procedures described above:

### 5-[2-(4-Chloro-phenylamino)-pyrimidin-4-yl]-3,4-dimethyl-3H-thiazol-2-one [23]

Light yellow solid; ¹H-NMR (DMSO-d₆) δ: 2.55 (s, 3H, CH₃), 3.29 (s, 3H, CH₃), 6.97 (d, 1H, *J* = 5.0 Hz, pyrimidinyl-H), 7.32 (d, 2H, *J* = 8.5 Hz, Ph-H), 7.76 (d, 2H, *J* = 9.0 Hz, Ph-H), 8.44 (d, 1H, *J*= 5.0 Hz, pyrimidinyl-H), 9.75 (br. s, 1H, NH).

### 5-[2-(4-Methoxy-phenylamino)-pyrimidin-4-yl]-3,4-dimethyl-3H-thiazol-2-one [24]

Light yellow solid; ¹H-NMR (DMSO-d₆) δ: 2.54 (s, 3H, CH₃), 3.28 (s, 3H, CH₃), 3.71 (s, 3H, CH₃), 6.86 (m, 3H, pyrimidinyl-H & Ph-H), 7.59 (d, 2H, *J*= 9.0 Hz, Ph-H), 8.37 (d, 1H, *J* = 5.0 Hz, pyrimidinyl-H), 9.39 (br. s, 1H, NH).

### 5-[2-(3-Hydroxy-phenylamino)-pyrimidin-4-yl]-3,4-dimethyl-3H-thiazol-2-one [25]

Light yellow solid; anal. RP-HPLC: t*_{R}*= 15.4 min (0 - 60 % MeCN in 0.1 % aq VF₃COOH over 20 min, 1 mL/min, purity > 95 %). ¹H-NMR (DMSO-d₆) δ: 2.55 (s, 3H, CH₃), 3.26 (s, 3H, CH₃), 6.36 (m, 1H, Ph-H), 6.90 (d, 1H, *J=* 5.5 Hz, pyrimidinyl-H), 7.03 (t, 1H, *J=* 8.5 Hz, Ph-H), 7.16 (m, 1H, Ph-H), 7.22 (s, 1H, Ph-H), 8.40 (d, 1H, *J=* 5.5 Hz, pyrimidinyl-H), 9.39 (br. s, 1H, NH).

### 5-[2-(4-Dimethylamino-phenylamino)-pyrimidin-4-yl]-3,4-dimethyl-3H-thiazol-2-one [26]

Yellow solid; anal. RP-HPLC: t*_{R}*=19.6 min (0 - 60 % MeCN in 0.1 % aq CF₃COOH over 20 min, 1 mL/min, purity > 95 %). ¹H-NMR (DMSO-d₆) δ. 2.83 (s, 3H, CH₃), 2.90 (s, 6H, CH₃), 3.08 (s, 3H, CH₃), 6.73 (m, 2H, Ph-H), 6.81 (d, 1H, *J=* 5.5 Hz, pyrimidinyl-H), 7.03 (m, 1H, Ph-H), 7.50 (m, 1H, Ph-H), 8.32 (d, 1H, *J*= 5.5 Hz, pyrimidinyl-H), 9.24 (br. s, 1H, NH).

### 5-[2-(4-Fluoro-3-nitro-phenylamino)-pyrimidin-4-yl] -3,4-dimethyl-3H-thiazol-2-one [28]

Brown solid; ¹H-NMR (DMSO-d₆) δ: 2.42 (s, 3H, CH₃), 2.81 (s, 3H, CH₃), 6.36 (m, 1H, Ph-H), 6.91 (d, 1H, *J* = 5.5 Hz, pyrimidinyl-H), 7.31 (m, 1H, Ph-H), 8.33 (m, 1H, Ph-H), 8.48 (d, 1H, *J*= 5.5 Hz, pyrimidinyl-H), 8.52 & 9.68 (br. s, 1H, NH).

### 3,4-Dimethyl-5-[2-(3-nitro-phenylamino)-pyrimidin-4-yl] -3H-thiazol-2-one [32]

Brown crystals. Anal. RP-HPLC: t*_{R}* = 17.8 min (0 - 60 % MeCN in 0.1 % aq CF₃COOH over 20 min, 1 mL/min, purity > 97%). ¹H-NMR (DMSO-d₆) δ: 2.42 (s, 3H, CH₃), 3.16 (s, 3H, CH₃), 6.92 (d, 1H, *J=* 5.0 Hz, pyrimidinyl-H), 7.42 (d, 1H, *J=* 8.0 Hz, Ph-H) 7.65 (m, 1H, Ph-H), 7.88 (m, 1H, Ph-H), 8.37 (d, 1H, *J*= 5.0Hz, pyrimidinyl-H), 8.72 (br. s, 1H, NH).

### 5-[2-(4-Fluoro-phenylamino)-pyrimidin-4-yl]-3,4-dimethyl-3H-thiazol-2-one [33]

Gray solid; ¹H-NMR (DMSO-d*₆*) δ: 2.92 (s, 3H, CH₃), 3.67 (s, 3H, CH₃), 7.32 (d, 1H, *J =* 5.0 Hz, pyrimidinyl-H), 7.51 (m, 2H, Ph-H), 8.11 (m, 2H, Ph-H), 8.80 (d, 1H, *J*= 5.0 Hz, pyrimidinyl-H).

### 3,4-Dimethyl-5-[2-(4 -methyl-3-nitro-phenylamino)-pyrimidin-4-yl]-3H-thiazol-2-one [36]

Yellow solid; ¹H-NMR (DMSO-d₆) δ: 2.44 (s, 3H, CH₃), 2.55 (s, 3H, CH₃), 3.27 (s, 3H, CH₃), 7.03 (d, 1H, *J* = 5.0 Hz, pyrimidinyl-H), 7.40 (t, 1H, *J*= 8.5 Hz, Ph-H), 7.84 (m, 1H, Ph-H), 8.48 (d, 1H, *J*= 5.0 Hz, pyrimidinyl-H), 8.59 (s, 1H, Ph-H), 9.99 (br. s, 1H, NH).

### 5-[2-(4-Fluoro-3-methyl-phenylamino)-pyrimidin-4-yl]-3,4-dimethyl-3H-thiazol-2-one [37]

Grey solid; ¹H-NMR (DMSO-d₆) δ: 2.21 (s, 3H, CH₃), 2.55 (s, 3H, CH₃), 3.26 (s, 3H, CH₃), 6.92 (d, 1H, *J*= 5.0 Hz, pyrimidinyl-H), 7.04 (t, 1H, *J*= 9.0 Hz, Ph-H), 7.48 (m, 1H, Ph-H), 7.68 (m, 1H, Ph-H), 8.40 (d, 1H, J= 5.5 Hz, pyrimidinyl-H), 9.54 (br. s, 1H, NH).

### Example 11

### 2-Chloro-4-guanidino-benzoic acid ethyl ester

A solution of 2-chloro-4-phenylguanidino-benzoic acid (1.17g, 4.0 mmol) in absolute EtOH (4 mL) was treated with conc. H₂SO₄ (1 mL). After refluxing for 2 h the reaction mixture was cooled and poured into ice water (5 mL). This solution was treated with ammonia solution (1 mL). The resulting precipitates were collected and washed with Et₂O to afford the title compound as an off white solid. ¹H-NMR (DMSO-d₆) δ: 1.31 (t, 3H, *J =* 7.1 Hz, CH₃), 4.31 (m, 2H, CH₂), 7.29 (m, 1H, Ph-H), 7.43 (s, 1H, Ph-H), 7.68 (br. s, 2H, NH₂) and 7.85 (m, 1H, Ph-H).

### 2-Chloro-4-[4-(4-methyl-2-methylamino-thiazol-5-yl)-pyrimidin-2-ylaminol-benzoic acid ethyl ester [29]

A mixture of 2-chloro-4-guanidino-benzoic acid ethyl ester (0.76 g, 3.0 mmol) and 3-Dimethylamino-1-(4-methyl-2-methylamino-thiazol-5-yl)-propenonc (0.43 g, 2.0 mmol) in 2-methoxylethanol (5 mL) was treated with NaOH 180 mg). After refluxing for 24 h the reaction mixture was concentrated. The resulting precipitates were collected and recrystallised from MeOH to afford the title compound (149 mg) as a gray solid. ¹H-NMR (DMSO-d₆) δ: 1.35 (t, 3H, *J*= 7.1 Hz, CH₃), 4.36 (m, 2H, CH₂), 6.94 (d, 1H, *J*= 5.5 Hz, pyrimidinyl-H), 7.41-7.51 (m, 2H, Ph-H), 7.90 (s, 1H, Ph-H), 9.36 (d, 1H, *J* = 5.5 Hz, pyrimidinyl-H) and 9.56 (br. s, 1H, NH).

### Example 12

### 1-(2-Amino-4-methyl-thiazol-5-yl)-ethanone

A mixture of thiourea (5.18 g, 0.068 mol) in dry MeOH (20 mL) was stirred and cooled on an ice bath. Pyridine (2 mL) was added, followed by 3-chloro-2,4-pentadione (9.15 g, 0.068 mol) dropwise. After completion of the addition the reaction mixture was allowed to warm to room temperature and stirring was continued for 4 h. The precipitates were filtered and washed with EtOAc to afford the title compound as a white solid.

### N'-[5-(3-Dimethylamino-acryloyl)-4-methyl-thiazol-2-yl]-N,N-dimethyl-formamidine

A solution of 1-(2-amino-4-methyl-thiazol-5-yl)-ethanone (3.35 g, 0.021 mol) in *N,N-*dimethylformamide dimethylacetal (10 mL) was refluxed under N₂ for 4 - 6h. The reaction mixture was evaporated to dryness. EtOAc was added to the residue and the precipitates were collected by filtration and were washed with EtOAc/PE (5:1, v/v) to afford the title compound as an orange solid (50 - 79 %). ¹H-NMR (CDCl₃) δ: 2.64 (s, 3H, CH₃), 3.08 (s, 6H, CH₃), 3.11 (s, 6H, CH₃), 5.35 (d, 1H, *J*= 12.2 Hz, CH), 7.67 (d, 1H, *J*= 12.2 Hz, CH), 8.23 (s, 1H, N=CH). DE MALDI-TOF MS: [M+H]⁺ = 267.49 (C₁₂H₁₈N₆OS requires 266.36).

### [4-(2-Amino-4-methyl-thiazol-5-yl)-pyrimidin-2-yl]-(3-nitro-phenyl)-amine [30]

A mixture of N-[5-(3-dimethylamino-acryloyl)-4-methyl-thiazol-2-yl] *N,N* dimethyl-formamidine (2.19 g, 8.2 mmol) and 3-nitrophenyl guanidine nitrate (2.00 g 8.2 mmol) in 2-methoxyethanol (10 mL) was treated with NaOH (0.33 g). After refluxing under N₂ for 20 h the reaction mixture was concentrated and purified by silica-gel chromatography using EtOAc/PE (7:1) to elute the title compound as a light-yellow solid (1.95 g, 72 %), which was then recrystallised from EtOAc/MeOH. ¹H-NMR (DMSO-d₆) δ: 3.13 (s, 3H, CH₃), 7.02 (d, 1H, *J*= 5.5 Hz, pyrimidinyl-H), 7.59 (m, 4H, Ph-H and NH₂), 7.82 (m, 1H, Ph-H), 8.16 (m, 1H, Ph-H), 8.44 (d, 1H, *J*= 5.5 Hz, pyrimidinyl-H), 8.86 (br. s, 1H, NH).

### Example 13

### [4-(2-Chloro-4-methyl-thiazol-5-yl)-pyrimidin-2-yl]-(3-nitro-phenyl)-amine [35]

A solution of [4-(2-methoxy 4-methyl-thiazol-5-yl)-pyrimidin-2-yl]-(3-nitro-phenyl)-amine (0.71 g, 2.0 mmol) in CHCl₃ (2 mL) and DMF (0.14 mL) was cooled on an ice bath and was treated with SOCl₂ (1.5 mL). The reaction mixture was warmed to room temperature and then heated at reflux for 1.5 h. The reaction mixture was concentrated, poured into ice water (3 mL), and extracted with CH₂Cl₂. The organic phase was washed with brine, dried on MgSO₄, and filtered. The solvent was evaporated and the residue was recrystallised from CH₂Cl₂ to afford the title compound (61 mg) as a light yellow solid. Anal. RP-HPLC: t*_{R}* = 22.7 min (0 - 60 % MeCN in 0.1 % aq CF₃COOH over 20 min, 1 mL/min, purity > 95 %). ¹H-NMR (DMSO-d₆) δ: 3.56 (s, 3H, CH₃), 6.61 (d, 1H, *J*= 5.5 Hz, pyrimidinyl-H), 7.31 (t, 1H, *J*= 8.2 Hz, Ph-H), 7.54 (m, 1H, Ph-H), 7.81 (m, 1H, Ph-H) and 8.32 (m, 2H, pyrimidinyl-H and Ph-H).

### Example 14

### 4-Acetyl-3,5-dimethyl-1H-pyrrole-2-carbonitrile

Chlorosulfonyl isocyanate (1.60 mL, 18.38 mmol) in acetonitrile (10 mL) was slowly added to a suspension of 1-(2,4-dimethyl-1*H*-pyrrol-3-yl)-ethanone (1.87 g, 13.63 mmol) in anhydrous acetonitrile (15 mL) and dimethylformamide (5.0 mL) at -5 °C. The reaction mixture was stirred for 2 h before allowing to warm to room temperature overnight. The reaction mixture was then extracted from ice/water (100 mL) into ethyl acetate (4 x 100 mL). The organics were combined, washed with brine, dried over anhydrous magnesium sulfate and concentrated to yield a light brown solid, which was re-crystallised from diethyl ether to afford the title compound (1.52 g, 72 %). ¹H-NMR (*d₆*-DMSO) δ: 2.29 (3H, s, CH₃), 2.34 (3H, s, CH₃), 2.41 (3H, s, COCH₃), 12.34 (1H, s, NH).

### 4 Acetyl-1,3,5-trimethyl-1H-pyrrole-2-carbonitrile

A solution of 4-Acetyl-3,5-dimethyl-1*H* pyrrole-2-carbonitrile (0.567 g, 3.5 mmol) in tetrahydrofuran (5 mL) was added to a cooled (0 °C) slurry of sodium hydride (0.168 g, 7.0 mmol) in tetrahydrofuran (5mL): The reaction mixture was heated to 40°C and methyl iodide (350 µL, 5.25 mmol) was added and the reaction mixture stirred for 2 h. After cooling, the reaction was quenched with saturated aqueous sodium carbonate solution (50 mL) and extracted into ethyl acetate (3 x 50 mL) before drying over anhydrous magnesium sulfate to afford the title compound as a yellow oil (0.443 g, 72 %) after removal of solvent. ¹H-NMR (*d₆*-DMSO) δ: 2.32 (3H, s, CH₃), 2.37 (3H, s, CH₃), 2.45 (3H, s; COCH₃), 3.58 (3H, s, NCH₃).

### 4-(3-Dimethylamino-acryloyl)-1,3,5-trimethyl-1H-pyrrole-2-carbonitrile

4-Acetyl-1,3,5-trimethyl-1H pyrrole-2-carbonitrile (0.352 g, 2 mmol) was taken up in *tert-*butoxybis(dimethylamino)methane (496 µL, 2.40 mmol) and heated at 75°C for 22 h. The reaction mixture was dissolved in methanol (10 mL) and concentrated to yield a dark coloured oil. After addition of diethyl ether (20 mL), the dark brown solid product (0.442 g, 95 %) was filtered off to afford the title compound. ¹H NMR (d*₆-*DMSO) δ: 2.20 (3H, s, CH₃), 2.32 (3H, s, CH₃), 2.80 & 3.07 (6H, s, N(CH₃)₂), 3.54 (3H, s, NCH₃), 5.17 (1H, d, C=CH, *J=* 12.5 Hz), 7.45 (1H, d, C=CH, *J=* 12.5 Hz) .

### 4-[2-(4-Dimethylamino-phenylamino)-pyrimidin-4-yl]-1,3,5-trimethyl-1H-pyrrole-2-carbonitrile [38]

4-(3-Dimethylamino-acryloyl)-1,3,5-trimethyl-1*H*-pyrrole-2-carbonitrile (0.115 g, 0.5 mmol), *N*-(4-dimethylamino-phenyl)-guanidine nitrate (0.120 g, 0.5 mmol), and potassium carbonate (0.139 g, 1.0 mmol) were combined in 2-methoxyethanol (4 mL) and the mixture was heated at 115 °C for 22 h. After cooling, the inorganics were filtered off and the filtrate was concentrated to dryness. The crude product was purified by silica gel column chromatography to afford the title compound (53 mg, 31 %). ¹H-NMR (d₆-DMSO) δ 2.27 (3H, s, CH₃), 2.42 (3H, s, CH₃), 2.83 (6H, s, N(CH₃)₂), 3.63 (3H, s, NCH₃), 6.68 (2H, d, ArH, *J* = 8.8 Hz), 6.71 (1H, d, ArH, *J =* 5.4 Hz), 7.51 (2H, d, ArH, *J =* 8.8 Hz), 8.35 (1H, d, ArH, J= 5.4 Hz), 9.11 (1H, s, NH). ESI-MS: m/z 347 (M⁺+1).

### 4-[2-(4-Dimethylamino-3-nitro-phenylamino)-pyrimidin-4-yl]-1,3,5-trimethyl-1H-pyrrole-2-carbonitrile [39]

4-(3-Dimethylamino-acryloyl)-1,3,5-trimethyl-1H-pyrrole-2-carbonitrile (0.115 g, 0.5 mmol), *N*-(4-dimethylamino-3-nitro-phenyl)-guanidine nitrate (0.143 g, 0.5 mmol), and potassium carbonate (0.139 g, 1.0 mmol) were combined in 2-methoxyethanol (4 mL) and the mixture was heated at 115 °C for 22 h. After cooling, the inorganics were filtered off and the filtrate was concentrated to dryness. The crude product was purified by silica gel column column to afford the title compound (76 mg, 39 %). ¹H-NMR (d*₆*-DMSO) δ: 2.29 (3H, s, CH₃), 2.43 (3H, s, CH₃), 2.74 (6H, s, N(CH₃)₂), 3.64 (3H, s, NCH₃), 6.84 (1H, d, ArH, *J =* 4.9 Hz), 7.23 (1H, d, ArH, *J*= 8.8 Hz), 7.78 (1H, dd, ArH, *J =* 8.8, 2.9 Hz), 8.38 (1H, d, ArH, *J =* 2.9 Hz), 8.45 (1H, d, ArH, *J =* 4.9 Hz), 9.66 (1H, s, NH). ESI-MS: m/z 392 (M⁺ + 1).

### Example 15

### GSK-3β kinase assay

GSK-3 was obtained from New England Biolabs (UK) Ltd., Hitchin, Herts. The recombinant enzyme was isolated from a strain of *E. coli* that carries a clone expressing GSK-3β derived from a rabbit skeletal muscle cDNA library [Wang, Q.M.; Fiol, C.J.; DePaoli-Roach, A.A.; Roach, P.J. J. Biol. Chem., 1994, 269, 14566]. Inhibition of GSK-3 function was assessed by measurement of phosphorylation of CREB phosphopeptide KRREILSRRPphosphoSYR in the presence of test compounds. Using a 96-well assay format, GSK3 (7.5U) was incubated for 30 min at 30 °C in a total volume of 25 µL in 20 mM MOPS pH 7.2, 25 mM β-glycerophosphate, 5 mM EGTA, 1 mM DTT, 1 mM Na₃VO₃, 40 µM CREB peptide, 15 mM MgCl₂ and 100 µM ATP (containing 0.25 µCi [γ-³²P]-ATP) in the presence of varying concentrations of test compound. The samples were transferred to 96-well p81 filter plates (Whatman Polyfiltronics, Kent, UK), and the plates were washed 4 times with 200 µL/well of 75 mM aq orthophosphoric acid. Scintillation liquid (50 µL) was added to each well, and incorporated radioactivity for each sample was determined using a scintillation counter (TopCount, Packard Instruments, Pangbourne, Berks, UK).

### CDK/cyclin kinase assays

Compounds were investigated for their CDK2/cyclin E, CDK2/cyclin A, CDK1/cyclin B, and CDK4/cyclin D1 inhibitory activity. His₆-tagged recombinant human cyclin-dependent kinases CDK1/cyclin B1, CDK2/cyclin E, CDK2/cyclin A, and CDK4 were expressed in sf9 insect cells using a baculovirus expression system. Recombinant cyclin D1 was expressed in *E. coli.* Proteins were purified by metal chelate affinity chromatography, to greater than 90 % homogeneity. Kinase assays were performed in 96-well plates using recombinant CDK/cyclins. Assays were performed in assay buffer (25 mM β-glycerophosphate, 20 mM MOPS, 5 mM EGTA, 1 mM DTT, 1 mM Na₃VO₃, pH 7.4), into which were added 2 - 4 µg of active enzyme with appropriate substrates (purified histone H1 for CDK1 and CDK2, recombinant GST-retinoblastoma protein (residues 773-928) for CDK4). The reaction was initiated by addition of Mg/ATP mix (15 mM MgCl₂ + 100 µM ATP with 30-50 kBq per well of [γ-³²P]-ATP) and mixtures incubated for 10 - 45 min, as required, at 30 °C. Reactions were stopped on ice, followed by filtration through p81 or GF/C filterplates (for CDK4) (Whatman Polyfiltronics, Kent, UK). After washing 3 times with 75 mM aq orthophosphoric acid, plates were dried, scintillant added and incorporated radioactivity measured in a scintillation counter (TopCount, Packard Instruments, Pangbourne, Berks, UK). Compounds for kinase assays. were made up as 10 mM stocks in DMSO and diluted into 10 % DMSO in assay buffer. Data was analysed using curve-fitting software (GraphPad Prism version 3.00 for Windows, GraphPad Software, San Diego California USA) to determine IC₅₀ values (concentration of test compound which inhibits kinase activity by 50 %.).

### Example 16

### Differentiation of L6 rat myocytes and 3T3 mouse adipocytes

Rat skeletal muscle myoblasts L6/G8.C5 were seeded at 2.4 x 10⁵ cells per 10 cm dish in DMEM 10 % foetal calf serum (FCS), containing penicillin/streptomycin. When 90 % confluence was reached the medium was exchanged with α MEM, supplemented with 2 % FCS and penicillin/streptomycin. Medium was refreshed every 48 hours and 4-7 days later the myocytes were formed.

Mouse pre-adipocytes 3T3-F442A were seeded at 9 x 10⁵ cells per 10 cm dish in DMEM 10 % FCS, containing penicillin/streptomycin. When 90 % confluent, the same medium was supplemented with 1 µg/mL insulin. After 3-5 days (when most cells were differentiated) insulin was removed and 4 days later the cells were ready to use.

### Glycogen synthase (GS) assay

Cells on 10 cm dishes (Human Embryonic Kidney (HEK) 293 cells, L6 rat myocytes, or 3T3 mouse adipocytes) were treated with different concentrations of GSK3-inhibitors or DMSO vehicle for 90 min. Incubation medium was removed and cells were washed with ice-cold phosphate-buffered saline (PBS) prior to lysis on ice in 50 mM HEPES, pH 7.5, 10 mM EDTA, 100 mM NaF, 5 mM DTT, protease inhibitor cocktail (Sigma). After a freeze/thaw cycle the samples are sonicated for 10 sec and centrifuged at 15,000 g for 10 min at 4 °C. Lysate supernatants were snap-frozen on liquid nitrogen and stored at -80 °C. Lysates were assayed for glycogen synthase activity in buffer (50 mM Tris-HCI, pH 7.8, 20 mM EDTA, 25 mM NaF, 5 mM DTT, 1% glycogen, 0.3 mM UDP-glucose and 0.06 µCi of [¹⁴C]-UDP-glucose in the presence of 0.1 or 10 mM glucose-6-phosphate. The reaction was carried out for 30 min at 30°C. 70 µL of the reaction mixture (total volume 90 µL) were transferred to a GFC 96-well filter plate (bottom sealed with foil), containing 140 µL 96 % ethanol. The GFC plate was incubated for 1 h on ice and than washed with 66 % ethanol. To each well 100 µL scintillant liquid was added and the radioactivity of the samples was measured using a scintillation counter (Topcount, HP). Data are expressed as -fold increase in glycogen synthase activity ratios over those of control samples.

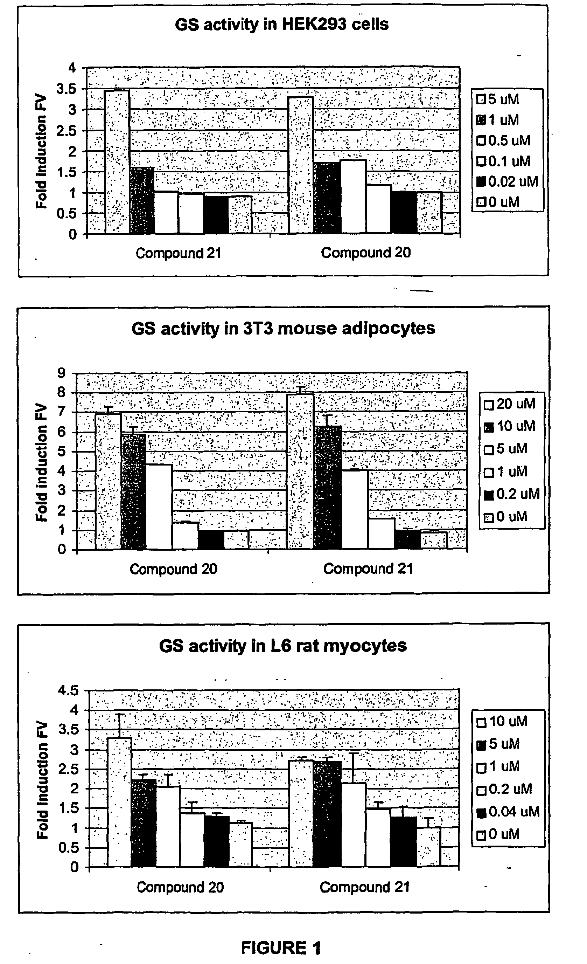

The biological activity of the exemplified compounds are summarised in Table 2. Activation of cellular glycogen synthase activity by example compounds [20] and [21] is shown in Figure 1.

Compounds [20] and [21] increased the activity of glycogen synthase in HEK293, rat myocyte, and mouse adipocyte cells, measured by the fractional reaction velocity of the enzyme (the ratio between the activity at 0.1 and 10 mM glucose-6 phosphate substrate concentrations). Both compounds increased the activity of GS in a dose-dependent manner. At 1 µM both compounds induced 2- and 4-fold activation of GS in myocytes and adipocytes, respectively.

### Example 18

### Oral glucose tolerance test (OGTT)

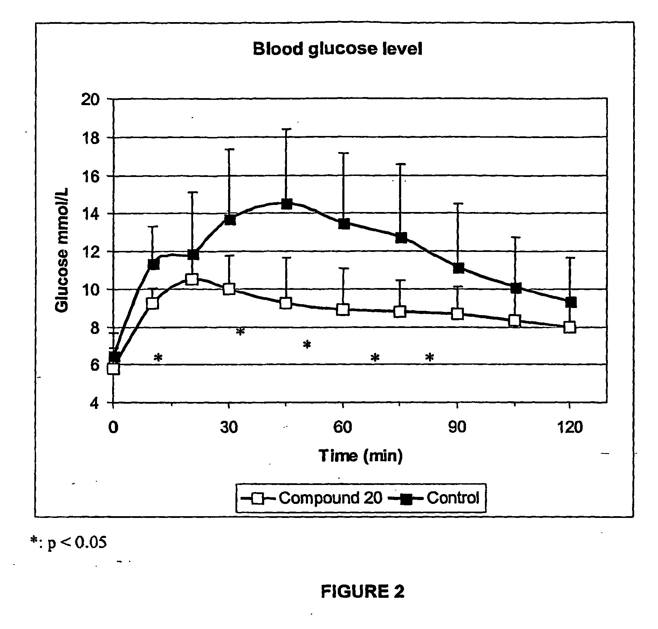

For the OGTT male ZDF fa/fa rats (Charles River, USA), 10-11 weeks old, were used. After 15 h fasting the animals were dosed intravenously with 5 mg/kg test compound in dosing vehicle, or with dosing vehicle (10 % DMSO, 90 % PEG-400) only at -270 and-30 min. At 0 min the rats were given 2 g/kg glucose by oral gavage. Plasma samples were taken before and every 15 min after the OGTT for determination of blood glucose. The results are shown in Figure 2.

Compound [20] improved the glucose tolerance in ZDF rats significantly. Compound [20] decreased the reactive and absolute AUC by 39 and 22 %, respectively. Insulin levels also decreased after treatment with compound [20], suggesting that the glucose lowering effect is due to the improvement of the insulin resistance rather than increase of the insulin level. This confirms the proposed mechanism of action for GSK3 inhibitors.

Various modifications and variations of the described methods of the invention will be apparent to those skilled in the art without departing from the scope and spirit of the invention. Although the invention has been described in connection with specific preferred embodiments, various modifications of the described modes for carrying out the invention which are obvious to those skilled in chemistry or related fields are intended to be within the scope of the following claims.

**Table 1: Exemplified compounds**

| **Number** | **Structure** | **Name** |
|---|---|---|
| **1** | 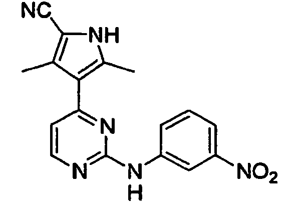 | 3,5-Dhnethyl-4-[2-(3-nitro-phenylamino)-pyrimidin-4-yl]-1H-pyrrole-2-carbonitrile |
| **2** | 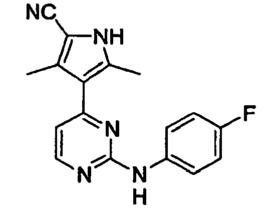 | 4-[2-(4-Fluoro-phenylamino)-pyri.midin-4-yl]-3,5-dimethyl-1 H-pyrrole-2-carbonitrile |
| **3** | 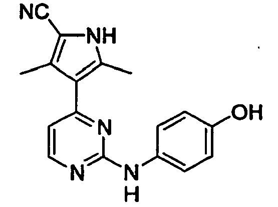 | 4-[2-(4-Hydroxy-phenylamino)-pyrimidin-4-yl]-3,5-dimethyl-1H-pyrrole-2-carbonitrile |
| **4** | 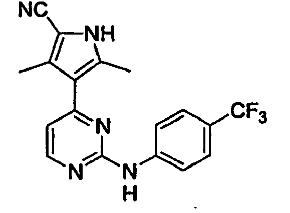 | 3,5-Dimethyl-4-[2-(4-trifluoromethyl-phenylami.no)-pyrimidin-4-yl]-1H-pyrrole-2-carbonitrile |
| **5** | 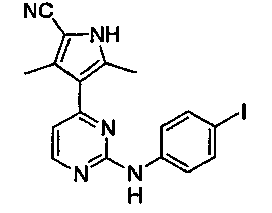 | 4-[2-(4-Iodo-phenylamino)-pyrimidin-4-yl]-3,5-dimethyl-1H-pyrrole-2-carbonitrile |
| **6** | 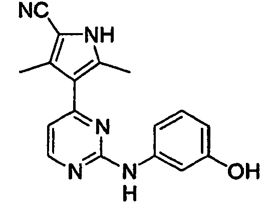 | 4-[2-(3-Hydroxy-phenylamino)-pyrimidin-4-yl]-3,5-dimethyl-1H-pyrrole-2-carbonitrile |
| **7** | 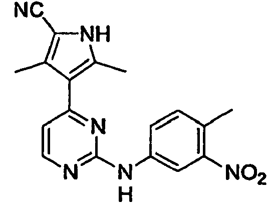 | 3,5-Dimethyl-4-[2-(4-methyl-3-nitro-phenylamino)-pyrimidin-4-yl]-1H-pyrrole-2-carbonitrile |
| **8** | 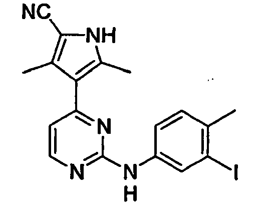 | 4-[2-(3-Iodo-4-methyl-phenylamino)-pyrimidin-4-yl]-3,5-dimethyl-1H-pyrrole-2-carbonitrile |
| **9** | 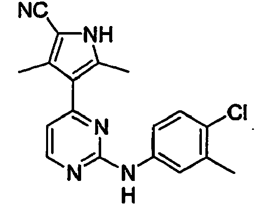 | 4-[2-(4-Chloro-3-methyl-phenylamino)-pyrimidin-4-yl]-3,5-dimethyl-1H-pyrrole-2-carbonitrile |
| **10** | 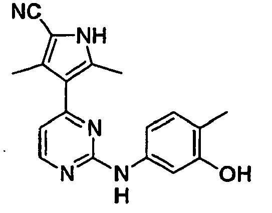 | 4-[2-(3-Hydroxy-4-methyl-phenylamino)-pyrimidin-4-yl]-3,5-dimethyl-1 H-pyaole-2-carbonitrile |
| **11** | 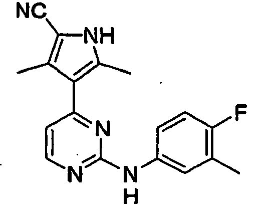 | 4-[2-(4-Fluoro-3-methyl-phenylamino)-pyrimidin-4-yl]-3,5-dimethyl-1H-pyrrole-2-carbonitrile |
| **12** | 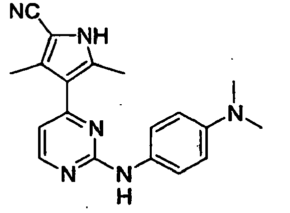 | 4-[2-(4-Dimethylamino-phenylamino)-pyrimidin-4- yl]-3,5-dimethyl-1H -pyrrole-2-carbonitrile |
| **13** | 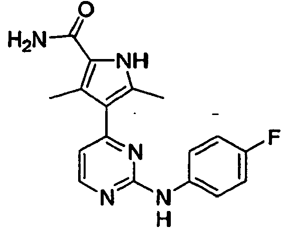 | 4-[2-(4-Fluoro-phenylamino)-pyrimidin-4-yl]-3,5-dimethyl-1H-pyirole-2-carboxylic acid amide |
| **14** | 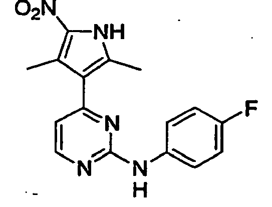 | [4-(2,4-Ditnethyl-5 -nitro- 1H-pyzrol-3-yl)-pyrimidin-2-yl]-(4-fluoro-phenyl)-amine |
| **15** | 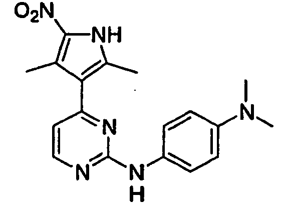 | N-[4-(2,4-Dimethyl-5-nitro-1H-pyrrol-3-yl)-pyrimidin-2-yl]-N',N'-dimethyl-benzene-1,4-diamine |
| **16** | 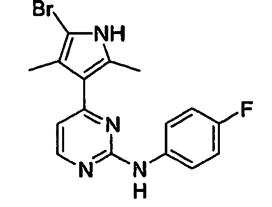 | [4-(5-Bromo-2,4-dimethyl-1H-pyrrol-3-yl)-pyrimidin-2-yl]-(4-fluoro-phenyl)-amine |
| **17** | 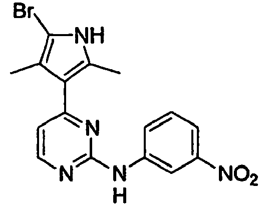 | [4-(5-Bromo-2,4-dimethyl-1H-pyrrol-3-yl)-pyrimidin-2-yl]-(3-nitro-phenyl)-amine |
| **18** | 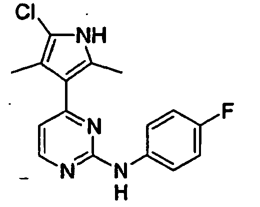 | [4-(5-Chloro-2,4-dimethyl-1H-pyrrol-3-yl)-pyrimidin-2-yl]-(4-fluoro-phenyl)-amine |
| **19** | 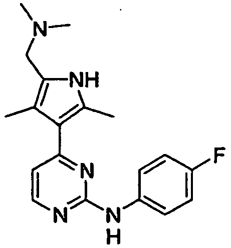 | [4-(5-Dimethylammomethyl-2,4-dimethyl-1H-pyrrol-3-yl)-pyrimidin-2-yl]-(4-fluoro-phenyl)-amine |
| **20** | 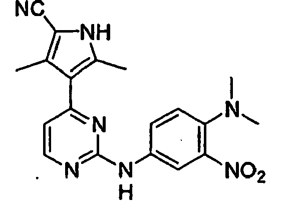 | 4-[2-(4-Dimethylamino-3-nitro-phenylamino)-pyrimidin-4-yl]-3,5-dimethyl-1H-pyaole-2-carbonitrile |
| **21** | 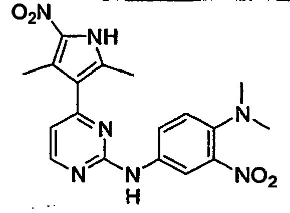 | N⁴-[4-(2,4-Dimethyl-5-nitro-1H-pyrrol-3-yl)-pyrimidin-2-yl]-N¹,N¹-dimethyl-2-nitro-ben zene-1,4-diamine |
| **22** | 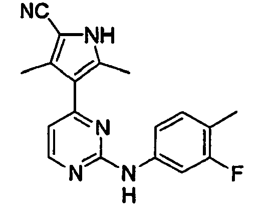 | 4-[2-(3-Fluoro-4-methyl-phenylamino)-pyrimidin-4-yl]-3,5-dimethyl-1H-pyrrole-2-carbonitrile |
| **23** | 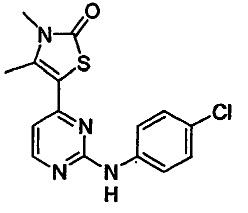 | 5-[2-(4-Chloro-phenylamino)-pyrimidin-4-yl]-3,4-dimethyl-3H-thiazol-2-one |
| **24** | 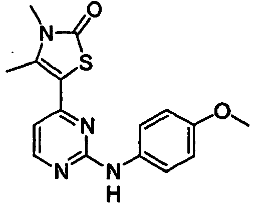 | 5-[2-(4-Methoxy-phenylamino)-pyrimidin-4-yl]-3,4-dimethyl-3H-thiazol-2-one |
| **25** | 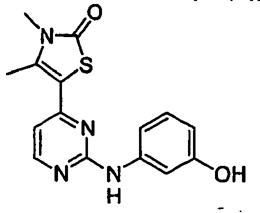 | 5-[2-(3-Hydroxy-phenylamino)-pyrimidin-4-yl]-3,4-dimethyl-3H-thiazol-2-one |
| **26** | 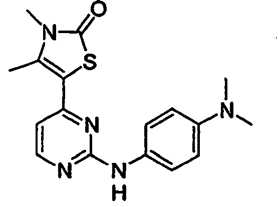 | 5-[2-(4-Dimethylamino-phenylamino)-pyrimidin-4-yl]-3,4-dimethyl-3H-thiazol-2-one |
| **27** | 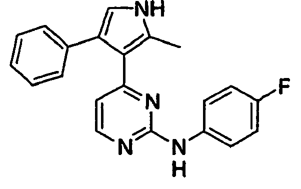 | (4-Fluoro-phenyl)-[4-(2-methyl-4-phenyl-1H-pyrrol-3-yl)-pyrimidin-2-yl]-amine |
| **28** | 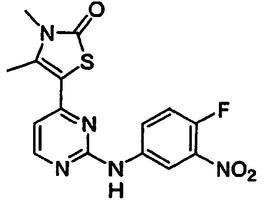 | 5-[2-(4-Fluoro-3-nitro-phenylamino)-pyrimidin-4-yl]-3,4-dimethyl-3H-thiazol-2-one |
| **29** | 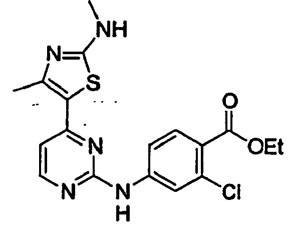 | 2-Chloro-4-[4-(4-methyl-2-methylamino-thiazol-5-yl)-pynmidin-2-ylamino]-benzoic acid ethyl ester |
| **30** | 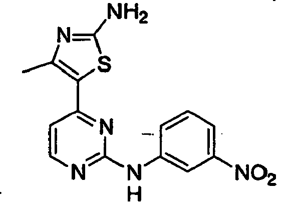 | [4-(2-Amino-4-methyl-thiazol-5-yl)-pyrimidin-2-yl]-(3-nitro-phenyl)-amine |
| **32** | 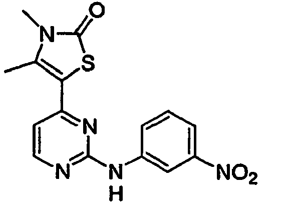 | 3,4-Dimethyl-5-[2-(3-nitro-phenylamino)-pyrimidin-4-yl]-3H-thiazol-2-one |
| **33** | 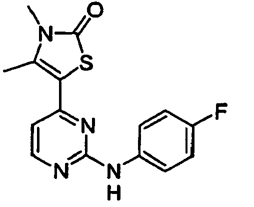 | 5-[2-(4-Fluoro-phenylamino)-pyrimidin-4-yl]-3,4-dimethyl-3H-thiazol-2-one |
| **34** | 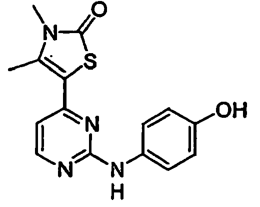 | 5-[2-(4-Hydroxy-phenylamino)-pyrimidin-4-yl]-3,4-dimethyl-3H-thiazol-2-one |
| **35** | 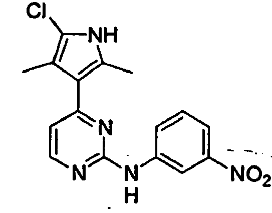 | [4-(5-Chloro-2,4-dimethyl-1H-pyrrol-3-yl)-pyrimidin-2-yl]-(3-nitro-phenyl)-amine |
| **36** | 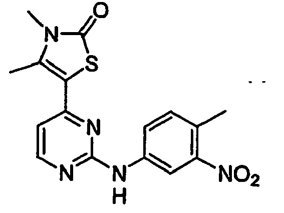 | 3,4-Dimethyl-5-[2-(4-methyl-3-nitro-phenylamino)-pyrimidin-4-yl]-3H-thiazol-2-one |
| **37** | 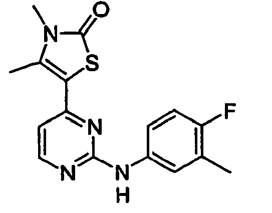 | 5-[2-(4-Fluoro-3-methyl-phenylamino)-pyrimidin-4-yl]-3,4-dimethyl-3H-thiazol-2-one |
| **38** | 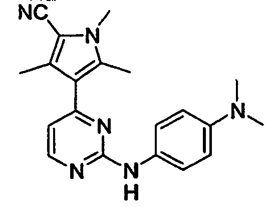 | 4-[2-(4-Dimethylamino-phenylamino)-pyrimidin-4-yl]-1,3,5-timethyl-1H-pyrrole-2-carbonitrile |
| **39** | 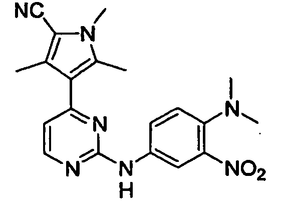 | 4-[2-(4-Dimethylamino-3-nitro-phenylamino)-pyrimidin-4-yl]-1,3,5-trimethyl-1H-pyrrole-2-carbonitrile |

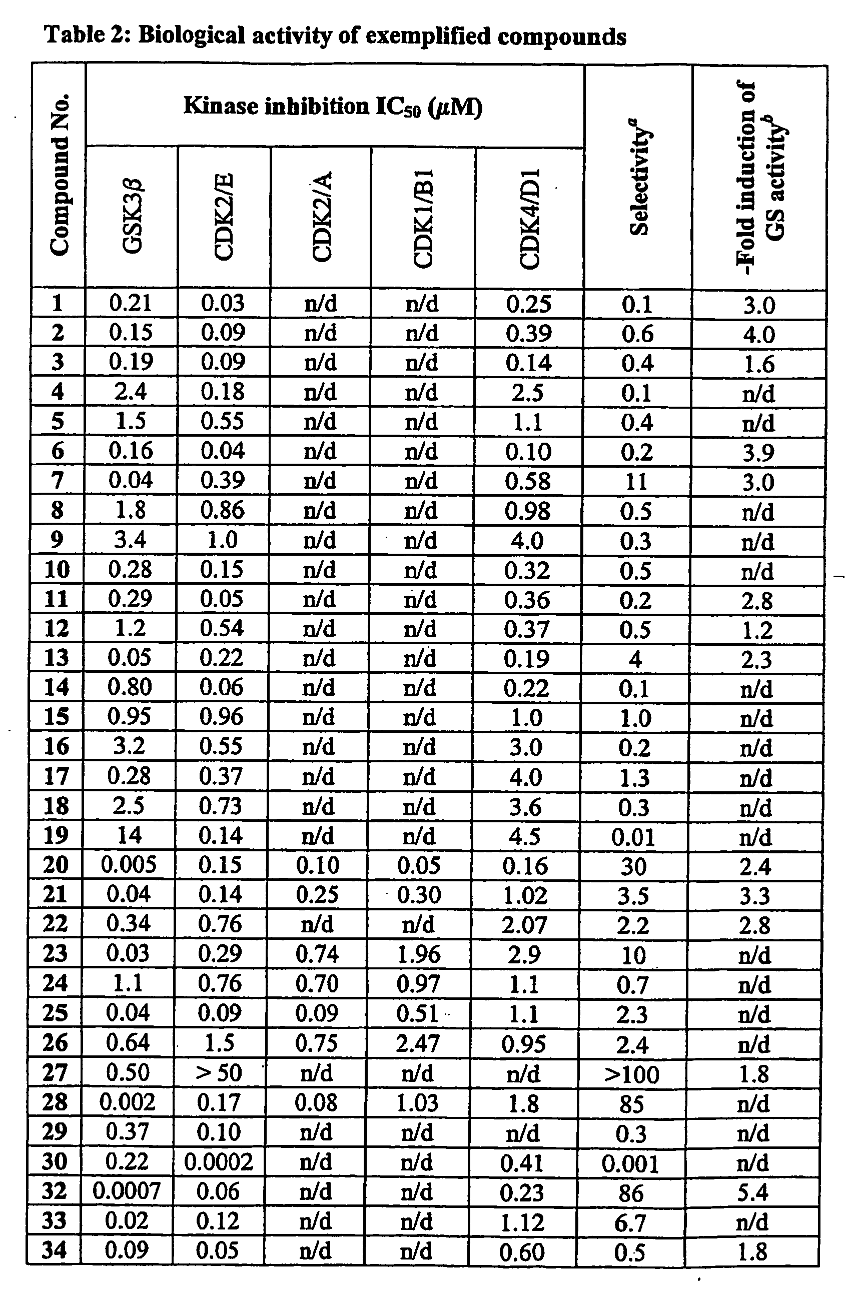

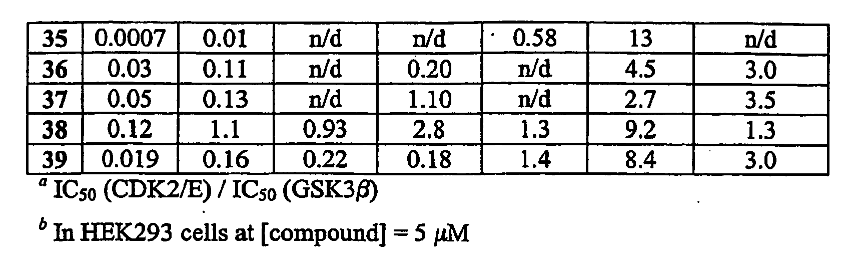

## Claims

1. Use of a compound of formula I, or a pharmaceutically acceptable salt thereof, 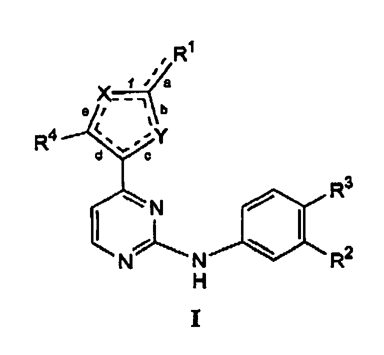 wherein
(A) one of X and Y is S, and the other is N; or
one of X and Y is NH or N-R⁵, and the other is C-R⁶;
"a" is a single bond;
"b", "c", "d", "e" and "f" are single or double bonds so as to form a heteroaryl ring;
R¹ is R⁷ with the proviso that R¹ is other than H or Me; or
(B) one of X and Y is S, and the other is NH or N-R⁵;
"a" and "d" are each double bonds;
"b", "c", "e" and "f" are each single bonds;
R¹ is oxo; and
R², R³, R⁴, R⁵, and R⁶ are each independently H or R⁷;
R⁷ is a group (CH₂)ₙ-R⁸, wherein n is 0,1,2, 3 or 4 and wherein R⁸ is selected from alkyl, aryl, heteroaryl, heterocycloalkyl, F, Cl, Br, I, CF₃, NO₂, CN, OH, O-alkyl, O-aryl, O-heteroaryl, O-hetcrocycloalkyl, CO-alkyl, CO-aryl, CO-heteroaryl, CO-heterocycloalkyl, COO-alkyl, NH₂, NH-alkyl, NH-aryl, N(alkyl)₂, NH-heteroaryl, NH-heterocycloalkyl, COOH, CONH₂, CONH-alkyl, CON(alkyl)₂, CONH-aryl, CONH-heteroaryl, CONH-heterocycloalkyl, SO₃H, SO₂-allcyl, SO₂-aryl, SO₂-heteroaryl, SO₂-heterocycloalkyl, SO₂NH₂, SO₂NH-alkyl, SO₂N(alkyl)₂, SO₂NH-aryl, SO₂NH-heteroaryl, or SO₂NH-heterocycloalkyl, wherein said alkyl, aryl, heteroaryl, and heterocycloalkyl groups are optionally substituted with one or more groups selected from halogeno, NO₂, OH, O-methyl, NH₂, COOH, CONH₂ and CF₃;
in the preparation of a medicament for treating diabetes.

2. Use according to claim 1 wherein said compound is of formula Ia, 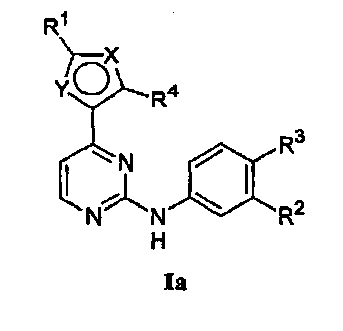 wherein
one of X and Y is N and the other is S; or
one of X and Y is NH or N-R⁵ and the other is C-R⁶;
R¹ is R⁷ with the proviso that R¹ is other than H or Me.
R¹⁻⁷ are as defined in claim 1.

3. Use according to claim 2 wherein X is N and Y is S; or X is NH or N-R⁵ and Y is C-R⁶.

4. Use according to claim 2 or claim 3 wherein:
R¹ is selected from CN, CONH₂, NO₂, halo, CH₂N(alkyl)₂, O-alkyl, NH₂ and NH-alkyl;
R² is selected from NO₂, H, OH, halo and alkyl;
R³ is selected from H, halo, OH, CF₃, alkyl, N(alkyl)₂, O-alkyl, heterocycloalkyl and COO-alkyl;
R⁴ is alkyl;
R⁵ is H or alkyl; and
R⁶ is alkyl.

5. Use according to any one of claims 2 to 4 wherein:
R¹ is selected from CN, CONH₂, NO₂, Br, Cl, OMe, CH₂NMe₂, NH₂ and NHMe;
R² is selected from NO₂, H, OH, I, Me, F and Cl;
R³ is selected from H, F, OH, CF₃, I, Me, Cl, NMe₂, OMe, morpholino and COOEt;
R⁴ is Me;
R⁵ is H or Me; and
R⁶ is Me.

6. Use according to any one of claims 2 to 5 wherein X is NH or N-R⁵ and Y is C-R⁶.

7. Use according to claim 6 wherein:
R¹ is selected from CN, CONH₂, NO₂, halo and CH₂N(alkyl)₂;
R² is selected from NO₂, H, OH, halo and alkyl;
R³ is selected from H, halo, OH, CF₃, alkyl and N(alkyl)₂;
R⁴ is alkyl;
R⁵ is H or alkyl; and
R⁶ is alkyl.

8. Use according to claim 7 wherein:
R¹ is selected from CN, CONH₂, NO₂, Br, Cl and CH₂NMe₂;
R² is selected from NO₂, H, OH, I, Me and F;
R³ is selected from H, F, OH, CF₃, I, Me, Cl and NMe₂;
R⁴ is Me;
R⁵ is H or Me; and
R⁶ is Me.

9. Use according to claim 8 wherein:
R¹ is CN or CONH₂;
R² is NO₂ or H; and
R³ is F or Me.

10. Use according to any one of claims 2 to 5 wherein X is N and Y is S.

11. Use according to claim 10 wherein:
R¹ is selected from halo, NH₂ and NH-alkyl;
R² is selected from NO₂, H, OH, halo and alkyl;
R³ is selected from H, halo, OH, alkyl, N(alkyl)₂, O-alkyl, heterocycloalkyl and
COO-alkyl; and
R⁴ is alkyl.

12. Use according to 11 wherein:
R¹ is selected from Cl, NH₂ and NHMe;
R² is selected from NO₂, H, OH, Me and Cl; and
R³ is selected from H, F, OH, Me, Cl, NMe₂, OMe, morpholino and COOEt; and
R⁴ is methyl.

13. Use according to claim 12 wherein:
R² is H or NO₂; and
R³ is Cl or F.

14. Use according to claim 1 wherein said compound is of formula Ib 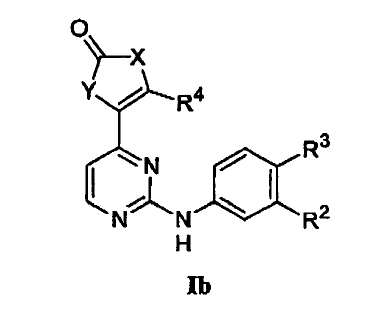 wherein one of X and Y is S, and the other is NH or N-R⁵; and
R²⁻⁵ are as defined in claim 1.

15. Use according to claim 14 wherein:
R² is selected from H, OH, NO₂ and alkyl;
R³ is selected from H, halogen, alkoxy, alkyl, N-(alkyl)₂ and OH; and
R⁴ and R⁵ are each independently alkyl.

16. Use according to claim 15 wherein:
R² is selected from H, OH, NO₂ and Me;
R³ is selected from H, Cl, F, OMe, Me, NMe₂ and OH; and
R⁴ and R⁵ are both Me.

17. Use according to any one of claims 1 to 16 wherein said compound of formula I is selected from the following:
3,5-Dimethyl-4-[2-(3-nitro-phenylamino)-pyrimidin-4-yl]-1H-pyrrole-2-carbonitrile **[1];**
4-[2-(4-Fluoro-phenylamino)-pyrimidin-4-yl]-3,5-dimethyl-1H-pyrrole-2-carbonitrile **[2];**
4-[2-(4-Hydioxy-phenylanuno)-pyrinudin-4-yl]-3,5-dimethyl-1H-pyrrole-2-carbonitrile **[3];**
3,5-Dimethyl-4-[2-(4-trifluoromethyl-phenylamino )-pyrimidin-4-yl]-1H-pyrrole-2-carbonilrile **[4];**
4-[2-(4-Iodo-phenylamino)-pyrimidin-4-yl]-3,5-dimethyl-1H-pyrrole-2-carbonitrile **[5];**
4-[2-(3-Hydroxy-phenylamino)-pyrimidin-4-yl]-3,5-dimethyl-1H-pyrrole-2-carbonitrile **[6];**
3,5-Dimethyl-4-[2-(4-methyl-3-nitro-phenylamino)-pyrimidin-4-yl]-1*H*-pyrrole-2-carbonatrile **[7]**;
4-[2-(3-Iodo-4-methyl-phenylamino)-pyrimidin-4-yl]-3,5-dimethyl-1*H*-pyrrole-2-carbonitrile **[8];**
4-[2-(4-Chloro-3-methyl-phenylamino)-pyrimidin-4-yl]-3,5-dimethyl-1H-pyrrole-2-carbonitrile **[9];**
4-[2-(3-Hydroxy-4-methyl-phenylamino)-pyrimidin-4-yl]-3,5-dimethyl-1*H*-pyrrole-2-carbonitrile **[10];**
4-[2-(4-Fluoro-3-methyl-phenylamino)-pyrimidin-4-yl]-3,5-dimethyl-1*H*- pyrrole-2-carbonitrile **[11];**
4-[2-(4-Dimethylamino-phenylamino)-pyrimidin4-yl]-3,5-dimethyl-1*H*-pyrrole-2-carbonitrile **[12]**;
4-[2-(4-Fluoro-phenylaniino)-pyrimidin4-yl]-3,5-dimethyl-1H-pyrrole-2-carboxylic acid amide **[13];**
[4-(2,4-Dimethyl-5-nitro-1H-pyrrol-3-yl)-pyrimidin-2-yl]-(4-fluoro-phenyl)-amine **[14];**
N-[4-(2,4-Dimethyl-5-nitro-1H-pyrrol-3-yl)-pyrimidin-2-yl]-N',N'-dimethyl-benzene-1,4-diamine **[15];**
[4-(5-Bromo-2,4-dimethyl-1H-pyrrol-3-yl)-pyrimidin-2-yl]-(4-fluoro-phenyl)-amine **[16];**
[4-(5-Bromo-2,4-dimethyl-1H-pyrrol-3-yl)-pyrimidin-2-yl]-(3-nitro-phenyl)-amme **[17];**
[4-(5-Chloro-2,4-dimethyl-1H-pyrrol-3-yl)-pyrimidin-2-yl]-(4-fluoro-phenyl)-amine **[18];**
[4-(5-Dimethylanunomethyl-2,4-dimethyl-1H-pyrrol-1H-3-yl)-pyrimidin-2-yl]-(4-fluorophenyl)-amine **[19];**
4-[2-(4-Dimethylamino-3-nitro-phenylamino)-pyrimidin-4-yl]-3,5-dimethyl-1H-pyrrole-2-carbonitrile **[20];**
N⁴-[4-(2,4-Dimethyl-5-nitro-1H-pyrrol-3-yl)-pyrimidin-2-yl]-N¹,N¹-dimethyl-2-nitrobenzene- 1,4-diamine **[21];**
4-[2-(3-Fluoro-4-methyl-phenylamino)-pyrimidin-4-yl]-3,5-dimethyl-1H-pyrrole-2-carbonitrile **[22];**
5-[2-(4-Chloro-phenylamino)-pyrimidin-4-yl]-3,4-dimethyl-3H-thiazol-2-one **[23];**
5-[2-(4-Methoxy-phenylamino)-pyrimidm-4-yl]-3,4-dimethyl-3H-thiazol-2-one **[24];**
5-(2-(3-Hydroxy-phenylamino)-pyrimidin-4-yl]-3,4-dimethyl-3H-thiazol-2-one **[25];**
5-[2-(4-Dimethylamino-phenylammo)-pyrimidin-4-yl]-3,4-dimethyl-3H-thiazol-2-one **[26];**
5-[2-(4-Fluoro-3-nitro-phenylamino)-pyrimidin-4-yl]-3,4-dimethyl-3H-thiazol-2-one **[28];**
2-Chloro-4-[4-(4-methyl-2-methylamino-thiazol-5-yl)-pyrimidin-2-ylamino]-benzoic acid ethyl ester **[29];**
[4-(2-Amino-4-methyl-thiazol-5-yl)-pyrimidin-2-yl]-(3-nitro-phenyl)-amine **[30];**
3,4-Dimethyl-5-[2-(3-nitro-phenylamino)-pyrimidin-4-yl]-3H-thiazol-2-one **[32];**
5-(2-(4-Fluom-phenylamino)-pyrimidin-4-yl]-3,4-dimethyl-3H-thiazol-2-one **[33];**
5-[2-(4-Hydroxy-phenylamino)-pyrimidin-4-y1]-3,4-dxmethyl-3H-thiazol-2-one **[34];**
[4-(2-Chloro-4-methyl-thiazol-5-yl)-pyrimidm-2-yl]-(3-nitro-phenyl)-amine **[35];**
3,4-Dimethyl-5-[2-(4-methyl-3-nitro-phenylamino)-pyrimidin-4-yl]-3H-thiazol-2-one **[36];**
(4-Fluoro-3-methyl-phenyl)-[4-(2-methoxy-4-methyl-thiazol-5-yl)-pyrimidin-2-yl]-amine **[37];**
4-[2-(4-Dimethylamino-phenylamino)-pyrimidin-4-yl]-1,3,5-trimethyl-1H-pyrrole-2-carbonitrile **[38];** and
4-[2-(4-Dimethylamino-3-nitro-phenylamino)-pyrimidin-4-yl]-1,3,5-trimethyl-1H-pyrrole-2-carbonitrile **[39].**

18. Use according to claim 17 wherein said compound is selected from the following:
3,5-Dimethyl-4-[2-(3-nitro-phenylamino)-pyrimidin-4-yl]-1H-pyrrole-2-carbonitrile **[1];**
4-[2-(4-Fluoro-phenylamino)-pyrimidin-4-yl]-3,5-dimethyl-1H-pyrrole-2-carbonitrile **[2];**
4-[2-(3-Hydroxy-phenylamino)-pyrimidin-4-yl]-3,5-dimethyl-1H-pynrole-2-carbonitnle **[6];**
3,5-Dimethyl-4-[2-(4-methyl-3-nitro-phenylamino)-pyrimidin-4-yl]-1H-pyrrole-2-carbonitrile **[7];**
4-[2-(4-Fluoro-3-methyl-phenylamino)-pyrimidin-4-yl]-3,5-dimethyl-1*H*-pyrrole-2-carbonitrile **[11];**
4-[2-(4-Dimethylamino-phenylamino)-pyrimidin-4-yl]-3,5-dimethyl-1*H*-pyrrole- 2-carbonitrile **[12];**
4-[2-(4-Fluoro-phenylmnino)-pyrimidin-4-yl]-3,5-dimethyl-1H-pyrrole-2-carboxylic acid amide **[13];**
4-(2-(3-Fluoro-4-methyl-phenylamino)-pyrimidin-a-yl]-3,5-dimethyl-1H-pyrrole-2-carbonitrile **[22];**
3,4-Dimethyl-5-(2-(3-nitro-phenylamino)-pyrimidin-4-yl]-3H-thiazol-2-one **[32];**
3-p-(4-Hydroxy-phenylamino)-pyrimidin-4-yl]-3,4-dimethyl-3H-thiazol-2-one **[34];**
3,4-Dimethyl-5-[2-(4-methyl-3-nitro-phenylamino)-pyrimidin-4-yl]-3H-thiazol-2-one **[36];**
5-[2-(4-Fluoro-3-methyl-phenylammo)-pyrimidin-4-yl]-3,4-dimethyl-3H-thiazol-2-one **[37];**
4-[2-(4-Dimethylamino-phenylamino)-pynmidin-4-yl]-1,3,5-trimethyl-1 H-pyrrole-2-carbonitrile **[38];** and
4-[2-(4-Dimethylamnno-3-nitro-phenylamino)-pyrimidin-4-yl]-1,3,5-trimethyl-1H-pyrrole-2-carbonitrile **[39].**

19. Use according to claim 17 wherein said compound is selected from the following:
3,5-Dimethyl-4-[2-(4-methyl-3-nitro-phenylamino)-pyrimidin-4-yl]-1H-pyrrole-2-carbonitrile **[7];**
4-[2-(4-Fluoxo-phenylamino)-pyrimidin-4-yl]-3,5-dimethyl-1H-pytxole-2-caxboxylic acid amide **[13];**
3,4-Dimethyl-5-(2-(3 -nitro-phenylamino)-pyrimidin-4-yl]-3H-thiazol-2-one **[32]**;
3,4-Dimethyl-5-[2-(4-methyl-3-nitro-phenylamino)-pyrimidin-4-yl]-3H-thiazol-2-one **[36];**
5-[2-(4-Fluoro-3-methyl-phenylamino)-pyrimidin4-yl]-3,4-dimethyl-3H-thiazol-2-one **[37];**
4-[2-(4-Dimethylamino-pheuylannino)-pyrimidin-4-yl]-1,3,5-trimethyl-1H-pyrrole-2-carbonitrile **[38];** and
4-[2-(4-Dimethylamino-3-nitro-phenylamino)-pyrimidin-4-yl]-1,3,5-tximethyl-1H-pyrrole-2-carbonitrile **[39].**

20. Use according to any preceding claim wherein the diabetes is Type II diabetes.

21. Use according to any preceding claim wherein said compound of formula I, or pharmaceutically acceptable salt thereof, is admixed with a pharmaceutically acceptable diluent, excipient or carrier.

22. Use according to any preceding claim wherein said compound of formula I is administered in combination with one or more other active agents.

## Patentansprüche

1. Verwendung einer Verbindung der Formel I oder eines pharmazeutisch verträglichen Salzes davon 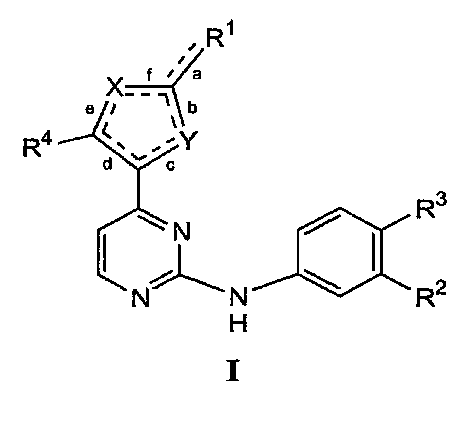 wobei
(A) eines unter X und Y S ist und das andere N ist, oder
eines unter X und Y NH oder N-R⁵ ist und das andere C-R⁶ ist,
"a" eine Einzelbindung ist,
"b", "c", "d", "e" und "f" Einzel- oder Doppelbindungen sind, um einen Heteroarylring auszubilden,
R¹ R⁷ ist mit der Maßgabe, dass R¹ anders als H oder Me ist, oder
(B) eines unter X und Y S ist, und das andere NH oder N-R⁵ ist,
"a" und "d" jeweils Doppelbindungen sind,
"b", "c", "e" und "f" jeweils Einzelbindungen sind,
R¹ eine Oxo-Gruppe ist und
R², R³, R⁴, R⁵ und R⁶ jeweils unabhängig voneinander H oder R⁷ sind,
R⁷ eine Gruppe (CH₂)ₙ-R⁸ ist, wobei n 0, 1, 2, 3 oder 4 ist, und wobei R⁸ ausgewählt ist unter Alkyl, Aryl, Heteroaryl, Heterocycloalkyl, F, Cl, Br, I, CF₃, NO₂, CN, OH, O-Alkyl, O-Aryl, O-Heteroaryl, O-Heterocycloalkyl, CO-Alkyl, CO-Aryl, CO-Heteroaryl, CO-Heterocycloalkyl, COO-Alkyl, NH₂, NH-Alkyl, NH-Aryl, N(Alkyl)₂, NH-Heteroaryl, NH-Heterocycloalkyl, COOH, CONH₂, CONH-Alkyl, CON(Alkyl)₂, CONH-Aryl, CONH-Heteroaryl, CONH-Heterocycloalkyl, SO₃H, SO₂-Alkyl, SO₂-Aryl, SO₂-Heteroaryl, SO₂-Heterocycloalkyl, SO₂NH₂, SO₂NH-Alkyl, SO₂N(Alkyl)₂. SO₂NH-Aryl, SO₂NH-Heteroaryl oder SO₂NH-Heterocycloalkyl, wobei die Alkyl-, Aryl-, Heteroaryl-und Heterocycloalkylgruppen wahlweise mit einer oder mehreren Gruppen substituiert sind, die ausgewählt ist unter Halogen, NO₂, OH, O-Methyl, NH₂, COOH, CONH₂ und CF₃,
bei der Herstellung eines Arzneimittels zur Behandlung von Diabtes.

2. Verwendung nach Anspruch 1, wobei die Verbindung die Formel la aufweist 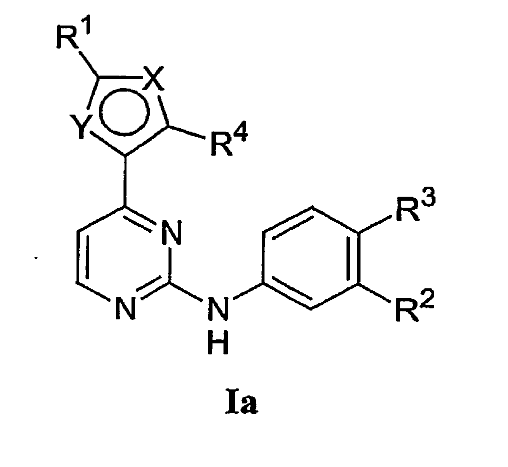 wobei
eines unter X und Y N ist und das andere S ist, oder
eines unter X und Y NH oder N-R⁵ ist und das andere C-R⁶ ist,
R¹ R⁷ ist mit der Maßgabe, dass R¹ anders als H oder Me ist,
R¹⁻⁷ wie in Anspruch 1 definiert sind.

3. Verwendung nach Anspruch 2, wobei X N ist und Y S ist oder X NH oder N-R⁵ ist und Y C-R⁶ ist.

4. Verwendung nach Anspruch 2 oder 3, wobei:
R¹ ausgewählt ist unter CN, CONH₂, NO₂ Halogen, CH₂N(Alkyl)₂, O-Alkyl, NH₂ und NH-Alkyl,
R² ausgewählt ist unter NO₂, H, OH, Halogen und Alkyl,
R³ ausgewählt ist unter H, Halogen, OH, CF₃, Alkyl, N(Alkyl)₂, O-Alkyl, Heterocycloalkyl und COO-Alkyl,
R⁴ Alkyl ist,
R⁵ H oder Alkyl ist, und
R⁶ Alkyl ist.

5. Verwendung nach einem der Ansprüche 2 bis 4, wobei:
R¹ ausgewählt ist unter CN, CONH₂, NO₂, Br, Cl, OMe, CH₂NMe₂, NH₂ und NHMe,
R² ausgewählt ist unter NO₂, H, OH, I, Me, F und Cl,
R³ ausgewählt ist unter H, F, OH, CF₃, I, Me, Cl, NMe₂. OMe, Morpholin und COOEt,
R⁴ Me ist,
R⁵ H oder Me ist, und
R⁶ Me ist.

6. Verwendung nach einem der Ansprüche 2 bis 5, wobei X NH oder N-R⁵ ist und Y C-R⁶ ist.

7. Verwendung nach Anspruch 6, wobei:
R¹ ausgewählt ist unter CN, CONH₂, NO₂, Halogen und CH₂N(Alkyl)₂,
R² ausgewählt ist unter NO₂, H, OH, Halogen und Alkyl,
R³ ausgewählt ist unter H, Halogen, OH, CF₃, Alkyl und N(Alkyl)₂,
R⁴ Alkyl ist,
R⁵ H oder Alkyl ist, und
R⁶ Alkyl ist.

8. Verwendung nach Anspruch 7, wobei:
R¹ ausgewählt ist unter CN, CONH₂, NO₂, Br, Cl und CH₂NMe₂,
R² ausgewählt ist unter NO₂, H, OH, I, Me und F,
R³ ausgewählt ist unter H, F, OH, CF₃, I, Me, Cl und NMe₂,
R⁴ Me ist,
R⁵ H oder Me ist, und
R⁶ Me ist.

9. Verwendung nach Anspruch 8, wobei:
R¹ ausgewählt ist unter CN oder CONH₂,
R² ausgewählt ist unter NO₂ oder H, und
R³ ausgewählt ist unter F oder Me.

10. Verwendung nach einem der Ansprüche 2 bis 5, wobei X N ist und Y S ist.

11. Verwendung nach Anspruch 10, wobei:
R¹ ausgewählt ist unter Halogen, NH₂ und NH-Alkyl,
R² ausgewählt ist unter NO₂, H, OH, Halogen und Alkyl,
R³ ausgewählt ist unter H, Halogen, OH, Alkyl, N(Alkyl)₂, O-Alkyl, Heterocycloalkyl und
COO-Alkyl, und
R⁴ Alkyl ist.

12. Verwendung nach Anspruch 11, wobei:
R¹ ausgewählt ist unter Cl, NH₂ und NHMe,
R² ausgewählt ist unter NO₂, H, OH, Me und Cl, und
R³ ausgewählt ist unter H, F, OH, Me, Cl, NMe₂, OMe, Morpholin und COOEt, und
R⁴ Methyl ist.

13. Verwendung nach Anspruch 12, wobei:
R² ausgewählt ist unter H oder NO₂, und
R³ Cl oder F ist.

14. Verwendung nach Anspruch 1, wobei die Verbindung die Formel Ib aufweist 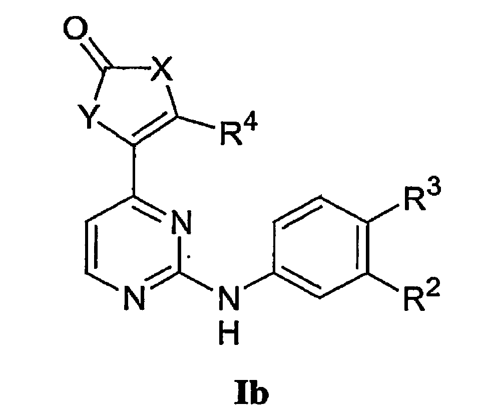 wobei eines unter X und Y S ist und das andere NH oder N-R⁵ ist, und
R²⁻⁵ wie in Anspruch 1 definiert sind.

15. Verwendung nach Anspruch 14, wobei:
R² ausgewählt ist unter H, OH, NO₂ und Alkyl,
R³ ausgewählt ist unter H, Halogen, Alkoxy, Alkyl, N-(Alkyl)₂ und OH, und
R⁴ und R⁵ jeweils unabhängig voneinander Alkyl sind.

16. Verwendung nach Anspruch 15, wobei:
R² ausgewählt ist unter H, OH, NO₂ und Me,
R³ ausgewählt ist unter H, Cl, F, OMe, Me, NMe₂ und OH, und
R⁴ und R⁵ beide Me sind.

17. Verwendung nach einem der Ansprüche 1 bis 16, wobei die Verbindung der Formel ausgewählt ist unter den folgenden:
3,5-Dimethyl-4-[2-(3-Nitrophenylamin)-pyrimidin-4-yl]-1H-pyrrol-2-carbonitril **[1],**
4-[2-(4-Fluorphenylamin)-pyrimidin-4-yl)-3,5-dimethyl-1H-pyrrol-2-carbonitril **[2],**
4-[2-(4-Hydroxyphenylamin)-pyrimidin-4-yl]-3,5-dimethyl-1H-pyrrol-2-carbonitril **[3],**
3,5-Dimethyl-4-[2-(4-trifluormethylphenylamin)-pyrimidin-4-yl]-1H-pyrrol-2-carbonitril **[4],**
4-[2-(4-lodphenylamin)-pyrimidin-4-yl]-3,5-dimethyl-1H-pyrrol-2-carbonitril **[5],**
4-[2-(3-Hydroxyphenylamin)-pyrimidin-4-yl]-3,5-dimethyl-1H-pyrrol-2-carbonitril **[6],**
3,5-Dimethyl-4-[2-(4-methyl-3-nitrophenylamin)-pyrimidin-4-yl]-1H-pyrrol-2-carbonitril **[7],**
4-[2-(3-lod-4-methylphenylamin)-pyrimidin-4-yl]-3,5-dimethyl-1*H*-pyrrol-2-carbonitril **[8],**
4-[2-(4-Chlor-3-Methylphenylamin)-pyrimidin-4-yl]-3,5-dimethyl-1H-pyrrol-2-carbonitril **[9],**
4-[2-(3-Hydroxy-4-methylphenylamin)-pyrimidin-4-yl]-3,5-dimethyl-1*H*-pyrrol-2-carbonitril **[10],**
4-[2-(4-Fluor-3-methylphenylamin)-pyrimidin-4-yl]-3,5-dimethyl-1*H*-pyrrol-2-carbonitril **[11],**
4-[2-(4-Dimethylaminphenylamin)-pyrimidin-4-yl]-3,5-dimethyl-1*H*-pyrrol-2-carbonitril **[12],**
4-[2-(4-Fluorphenylamin)-pyrimidin-4-yl]-3,5-dimethyl-1H-pyrrol-2-carbonsäureamid **[13],**
[4-(2,4-Dimethyl-5-nitro-1H-pyrrol-3-yl)-pyrimidin-2-yl]-(4-fluorphenyl)-amin **[14],**
N-[4-(2,4-Dimethyl-5-nitro-1H-pyrrol-3-yl)-pyrimidin-2-yl]-N',N'-dimethylbenzen-1,4-diamin **[15],**
[4-(5-Brom-2,4-dimethyl-1H-pyrrol-3-yl)-pyrimidin-2-yl]-(4-fluorphenyl)-amin **[16],**
[4-(5-Brom-2,4-dimethyl-1H-pyrrol-3-yl)-pyrimidin-2-yl]-(3-nitrophenyl)-amin **[17],**
[4-(5-Chlor-2,4-dimethyl-1H-pyrrol-3-yl)-pyrimidin-2-yl]-(4-fluorphenyl)-amin **[18],**
[4-(5-Dimethylaminmethyl-2,4-dimethyl-1H-pyrrol-3-yl)-pyrimidin-2-yl]-(4-fluorphenyl)-amin **[19],**
4-[2-(4-Dimethylamin-3-nitro-phenylamin)-pyrimidin-4-yl]-3,5-dimethyl-1H-pyrrol-2-carbonitril **[20],**
N⁴-[4-(2,4-Dimethyl-5-nitro-1H-pyrrol-3-yl)-pyrimidin-2-yl]-N¹,N¹-dimethyl-2-nitro-benzen-1,4-diamin **[21],**
4-[2-(3-Fluor-4-methylphenylamin)-pyrimidin-4-yl]-3,5-dimethyl-1H-pyrrol-2-carbonitril **[22],**
5-[2-(4-Chlorphenylamin)-pyrimidin-4-yl]-3,4-dimethyl-3H-thiazol-2-on **[23],**
5-[2-(4-Methoxyphenylamin)-pyrimidin-4-yl]-3,4-dimethyl-3H-thiazol-2-on **[24],**
5-[2-(3-Hydroxyphenylamin)-pyrimidin-4-yl]-3,4-dimethyl-3H-thiazol-2-on **[25],**
5-[2-(4-Dimethylaminphenylamin)-pyrimidin-4-yl]-3,4-dimethyl-3H-thiazol-2-on **[26],**
5-[2-( 4-Fluor -3-nitrophenylamin)-pyrimidin-4-yl]-3,4-dimethyl-3H-thiazol-2-on **[28],**
2-Chlor-4-[4-(4-methyl-2-methylaminthiazol-5-yl)-pyrimidin-2-ylamin]-benzoesäureethylester **[29],**
[4-(2-Amin-4-methylthiazol-5-yl)-pyrimidin-2-yl]-3-(nitrophenyl)-amin **[30],**
3,4-Dimethyl-5-[2-(3-nitrophenylamin)-pyrimidin-4-yl]-3H-thiazol-2-on **[32],**
5-[2-4-Fluorphenylamin)-pyrimidin-4-yl]-3,4-dimethyl-3H-thiazol-2-on **[33],**
5-[2-4-Hydroxyphenylamin)-pyrimidin-4-yl]-3,4-dimethyl-3H-thiazol-2-on **[34],**
[4-(2-Chlor-4-methylthiazol-5-yl)-pyrimidin-2-yl]-(3-nitrophenyl)-amin **[35],**
3,4-Dimethyl-5-[2-(4-methyl-3-nitrophenylamin)-pyrimidin-4-yl]-3H-thiazol-2-on **[36],**
(4-Fluor-3-methylphenyl)-[4-(2-methoxy-4-methylthiazol-5-yl)-pyrimidin-2-yl]-amin **[37],**
4-[2-(4-Dimethylaminphenylamin)-pyrimidin-4-yl]-1,3,5-trimethyl-1H-pyrrol-2-carbonitril **[38]** und
4-[2-(4-Dimethylamin-3-nitrophenylamin)-pyrimidin-4-yl]-1,3,5-trimethyl-1H-pyrroi-2-carbonitril **[39].**

18. Verwendung nach Anspruch 17, wobei die Verbindung ausgewählt ist unter den folgenden:
3,5-Dimethyl-4-[2-(3-Nitrophenylamin)-pyrimidin-4-yl]-1H-pyrrol-2-carbonitril **[1],**
4-[2-(4-Fluorphenylamin)-pyrimidin-4-yl)-3,5-dimethyl-1H-pyrrol-2-carbonitril **[2],**
4-[2-(3-Hydroxyphenylamin)-pyrimidin-4-yl]-3,5-dimethyl-1H-pyrrol-2-carbonitril **[6],**
3,5-Dimethyl-4-[2-(4-methyl-3-nitrophenylamin)-pyrimidin-4-yl]-1H-pyrrol-2-carbonitril **[7],**
4-[2-(4-Fluor-3-methylphenylamin)-pyrimidin-4-yl]-3,5-dimethyl-1H-pyrrol-2-carbonitril **[11],**
4-[2-(4-Dimethylaminphenylamin)-pyrimidin-4-yl]-3,5-dimethyl-1H-pyrrol-2-carbonitril **[12],**
4-[2-(4-Fluorphenylamin)-pyrimidin-4-yl]-3,5-dimethyl-1H-pyrrol-2-carbonsäureamid **[13],**
4-[2-(3-Fluor-4-methylphenylamin)-pyrimidin-4-yl]-3,5-dimethyl-1H-pyrrol-2-carbonitril **[22],**
3,4-Dimethyl-5-[2-(3-nitrophenylamin)-pyrimidin-4-yl]-3H-thiazol-2-on **[32],**
5-[2-4-Hydroxyphenylamin)-pyrimidin-4-yl]-3,4-dimethyl-3H-thiazol-2-on **[34],**
3,4-Dimethyl-5-[2-(4-methyl-3-nitrophenylamin)-pyrimidin-4-yl]-3H-thiazol-2-on **[36],**
5-[2-(4-Fluor-3-methylphenylamin)-pyrimidin-4-yl]-3,4-dimethyl-3H-thiazol-2-on **[37],**
4-[2-(4-Dimethylaminphenylamin)-pyrimidin-4-yl]-1,3,5-trimethyl-1H-pyrrol-2-carbonitril **[38],** und
4-[2-(4-Dimethylamin-3-nitrophenylamin)-pyrimidin-4-yl]-1,3,5-trimethyl-1H-pyrrol-2-carbonitril **[39].**

19. Verwendung nach Anspruch 17, wobei die Verbindung ausgewählt ist unter den folgenden:
3,5-Dimethyl-4-[2-(4-methyl-3-nitrophenylamin)-pyrimidin-4-yl]-1H-pyrrol-2-carbonitril **[7],**
4-[2-(4-Fluorphenylamin)-pyrimidin-4-yl]-3,5-dimethyl-1H-pyrrol-2-carbonsäureamid **[13],**
3,4-Dimethyl-5-[2-(3-nitrophenylamin)-pyrimidin-4-yl]-3H-thiazol-2-on **[32],**
3,4-Dimethyl-5-[2-(4-methyl-3-nitrophenylamin)-pyrimidin-4-yl]-3H-thiazol-2-on **[36],**
5-[2-(4-Fluor-3-methylphenylamin)-pyrimidin-4-yl]-3,4-dimethyl-3H-thiazol-2-on **[37],**
4-[2-(4-Dimethylaminphenylamin)-pyrimidin-4-yl]-1,3,5-trimethyl-1H-pyrrol-2-carbonitril **[38]** und
4-[2-(4-Dimethylamin-3-nitrophenylamin)-pyrimidin-4-yl]-1,3,5-trimethyl-1H-pyrrol-2-carbonitril **[39].**

20. Verwendung nach einem der vorangehenden Ansprüche, wobei der Diabetes vom Typ II ist.

21. Verwendung nach einem der vorangehenden Ansprüche, wobei die Verbindung der Formel | oder ein pharmazeutisch verträgliches Salz davon mit einem pharmazeutisch verträglichen Verdünnungsmittel, Hilfsstoff oder Träger vermischt ist.

22. Verwendung nach einem der vorangehenden Ansprüche, wobei die Verbindung der Formel I in Kombination mit einem oder mehreren anderen aktiven Mitteln verabreicht wird.

## Revendications

1. Utilisation d'un composé de formule I, ou d'un de ses sels pharmaceutiquement acceptables, 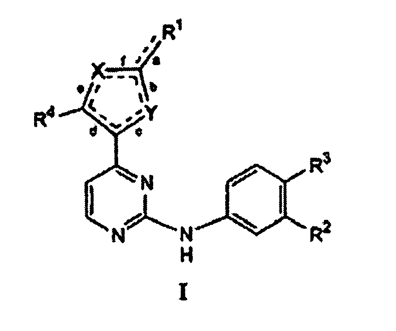 dans laquelle
(A) un de X et Y représente S et l'autre représente N ; ou un de X et Y représente un groupe NH ou N-R⁵ et l'autre représente un groupe C-R⁶ ;
"a" représente une liaison simple;
"b", "c", "d", "e" et "f" représentent des liaisons simples ou doubles de manière à former un noyau hétéroaryle ;
R¹ représente un groupe R⁷ sous réserve que R¹ soit autre que H ou un groupe Me ; ou
(B) un de X et Y représente S et l'autre représente un groupe NH ou N-R⁵ ;
"a" et "d" représentent chacun une double liaison;
"b", "c", "e" et "f" représentent chacun une liaison simple;
R¹ représente un groupe oxo ; et
R², R³, R⁴, R⁵ et R⁶ représentent chacun indépendamment H ou un groupe R⁷;
R⁷ représente un groupe (CH₂)ₙ-R⁸ dans lequel n est égal à 0, 1, 2, 3 ou 4 et dans lequel R³ est choisi entre des groupes alkyle, aryle, hétéroaryle, hétérocycloalkyle, F, Cl, Br, I, CF₃, NO₂, CN, OH, O-alkyle, O-aryle, O-hétéroaryle, O-hétérocycloalkyle, CO-alkyle, CO-aryle, CO-hétéroaryle, CO-hétérocycloalkyle, COO-alkyle, NH₂, NH-alkyle, NH-aryle, N(alkyle)₂, NH-hétéroaryle, NH-hétérocycloalkyle, COOH, CONH₂, CONH-alkyle, CON(alkyle)₂, CONH-aryle, CONH-hétéroaryle, CONH-hétérocycloalkyle, SO₃H, SO₂-alkyle, SO₂-aryle, SO₂-hétéroaryle, SO₂-hétérocycloalkyle, SO₂NH₂, SO₂NH-alkyle, SO₂N(alkyle)z, SO₂NH-aryle, SO₂NH-hétéroaryle et SO₂NH-hétérocycloalkyle, dans lesquels lesdits groupes alkyle, aryle, hétéroaryle et hétérocycloalkyle sont facultativement substitués avec un ou plusieurs groupes choisis entre des groupes halogéno, NO₂, OH, O-méthyle, NH₂, COOH, CONH₂ et CF₃ ;
dans la préparation d'un médicament destiné au traitement du diabète.

2. Utilisation suivant la revendication 1, dans laquelle ledit composé est un composé de formule Ia, 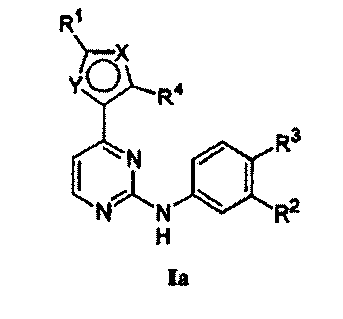 dans laquelle
un de X et Y représente N et l'autre représente S; ou
un de X et Y représente un groupe NH ou N-R⁵ et l'autre représente un groupe C-R⁶;
R¹ représente un groupe R⁷, sous réserve que R¹ soit autre que H ou un groupe Me.
Les groupes R¹⁻⁷ répondent aux définitions figurant dans la revendication 1.

3. Utilisation suivant la revendication 2, dans laquelle X représente N et Y représente S ; ou X représente un groupe NH ou N-R⁵ et Y représente un groupe C-R⁶.

4. Utilisation suivant la revendication 2 ou la revendication 3, dans laquelle :
R¹ est choisi entre des groupes CN, CONH₂, NO₂, halogéno, CH₂N(alkyle)₂, O-alkyle, NH₂ et NH-alkyle ;
R² est choisi entre des groupes NO₂, H, OH, halogéno et alkyle ;
R³ est choisi entre des groupes H, halogéno, OH, CF₃, alkyle, N(alkyle)₂, O-alkyle, hétérocycloalkyle, et COO-alkyle ;
R⁴ représente un groupe alkyle ;
R⁵ représente H ou un groupe alkyle ; et
R⁶ représente un groupe alkyle.

5. Utilisation suivant l'une quelconque des revendications 2 à 4, dans laquelle:
R¹ est choisi entre des groupes CN, CONH₂, NO₂, Br, Cl, OMe, CH₂NMe₂, NH₂ et NHMe ;
R² est choisi entre des groupes NO₂, H, OH, I, Me, F et Cl ;
R³ est choisi entre des groupes H, F, OH, CF₃, I, Me, Cl, NMe₂, OMe, morpholino et COOEt ;
R⁴ représente un groupe Me ;
R⁵ représente H ou un groupe Me ; et
R⁶ représente un groupe Me.

6. Utilisation suivant l'une quelconque des revendications 2 à 5, dans laquelle X représente un groupe NH ou N-R⁵ et Y représente un groupe C-R⁶.

7. Utilisation suivant la revendication 6, dans laquelle:
R¹ est choisi entre des groupes CN, CONH₂, NO₂, halogéno et CH₂N(alkyle)₂ ;
R² est choisi entre des groupes NO₂, H, OH, halogéno et alkyle;
R³ est choisi entre des groupes H, halogéno, OH, CF₃, alkyle et N(alkyle)₂ ;
R⁴ représente un groupe alkyle ;
R⁵ représente H ou un groupe alkyle ; et
R⁶ représente un groupe alkyle.

8. Utilisation suivant la revendication 7, dans laquelle :
R¹ est choisi entre des groupes CN, CONH₂, NO₂, Br, Cl et CH₂NMe₂ ;
R² est choisi entre des groupes NO₂ H, OH, I, Me et F ;
R³ est choisi entre des groupes H, F, OH, CF₃, I, Me, Cl et NMe₂ ;
R⁴ représente un groupe Me ;
R⁵ représente H ou un groupe Me ; et
R⁶ représente un groupe Me.

9. Utilisation suivant la revendication 8, dans laquelle :
R¹ représente un groupe CN ou CONH₂ ;
R² représente un groupe NO₂ ou H ; et
R³ représente un groupe F ou Me.

10. Utilisation suivant l'une quelconque des revendications 2 à 5, dans laquelle X représente N et Y représente S.

11. Utilisation suivant la revendication 10, dans laquelle:
R¹ est choisi entre des groupes halogéno, NH₂ et NH-alkyle;
R² est choisi entre des groupes NO₂, H, OH, halogéno et alkyle;
R³ est choisi entre H, des groupes halogéno, OH, alkyle, N(alkyle)₂, O-alkyle, hétérocycloalkyle et COO-alkyle ; et
R⁴ représente un groupe alkyle.

12. Utilisation suivant la revendication 11, dans laquelle :
R¹ est choisi entre des groupes Cl, NH₂ et NHMe ;
R² est choisi entre des groupes NO₂ H, OH, Me et Cl ;
R³ est choisi entre des groupes H, F, OH, Me, Cl, NMe₂, OMe, morpholino et COOEt ; et
R⁴ représente un groupe méthyle.

13. Utilisation suivant la revendication 12, dans laquelle :
R² représente H ou un groupe NO₂ ; et
R³ représente un groupe Cl ou F.

14. Utilisation suivant la revendication 1, dans laquelle ledit composé répond à la formule Ib 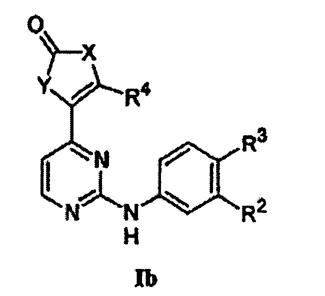 dans laquelle un de X et Y représente S et l'autre représente un groupe NH ou N-R⁵ ; et
les groupes R²⁻⁵ sont tels que définis dans la revendication 1.

15. Utilisation suivant la revendication 14, dans laquelle:
R² est choisi entre H, des groupes OH, NO₂ et alkyle ;
R³ est choisi entre H, des groupes halogéno, alkoxy, alkyle, N-(alkyle)₂ et OH ; et
R⁴ et R⁵ représentent chacun indépendamment un groupe alkyle.

16. Utilisation suivant la revendication 15, dans laquelle :
R² est choisi entre H, des groupes OH, NO₂ et Me ;
R³ est choisi entre H, des groupes Cl, F, OMe, Me, NMe₂ et OH ; et
R⁴ et R⁵ représentent l'un et l'autre un groupe Me.

17. Utilisation suivant l'une quelconque des revendications 1 à 16, dans laquelle ledit composé de formule I est choisi parmi les suivants :
3,5-diméthyl-4-[2-(3-nitro-phénylamino)-pyrimidine-4-yl] -1H-pyrrole-2-carbonitrile [1] ;
4-[2-(4-fluoro-phénylamino)-pyrimidine-4-yl]-3,5-diméthyl-1H-pyrrole-2-carbonitrile [2];
4-[2-(4-hydroxy-phénylamino)-pyrimidine-4-yl]-3,5-diméthyl-1H-pyrrole-2-carbonitrile [3] ;
3,5-diméthyl-4-[2-(4-trifluorométhyl-phénylamino)-pyrimidine-4-yl]-1H-pyrrole-2-carbonitrile [4] ;
4-[2-(4-iodo-phénylamino)-pyrimidine-4-yl]-3,5-diméthyl-1H-pyrrole-2-carbonitrile [5] ;
4-[2-(3-hydroxy-phénylamino)-pyrimidine-4-yl]-3,5-diméthyl-1H-pyrrole-2-carbonitrile [6] ;
3,5-diméthyl-4-[2-(4-méthyl-3-nitro-phénylamino)-pyrimidine-4-yl)-1H-pyrrole-2-carbonitrile [7] ;
4-[2-(3-iodo-4-méthyl-phénylamino)-pyrimidine-4-yl]-3,5-diméthyl-1H-pyrrole-2-carbonitrile [8] ;
4-[2-(4-chloro-3-méthyl-phénylamino)-pyrimidine-4-yl]-3,5-diméthyl-1H-pyrrole-2-carbonitrile [9] ;
4-[2-(3-hydroxy-4-méthyl-phénylamino)-pyrimidine-4-yl]-3,5-diméthyl-1*H*-pyrrole-2-carbonitrile [10] ;
4-[2-(4-fluoro-3-méthyl-phénylamino)-pyrimidine-4-yl]-3,5-diméthyl-1*H*-pyrrole-2-carbonitrile [11] ;
4-[2-(4-diméthylamino-phénylamino)-pyrimidine-4-yl]-3,5-diméthyl-1*H*-pyrrole-2-carbonitrile [12] ;
amide d'acide 4-[2-(4-fluoro-phénylamino)-pyrimidine-4-yl]-3,5-diméthyl-1H-pyrrole-2-carboxylique [13] ;
[4-(2,4-diméthyl-5-nitro-1H-pyrrole-3-yl)-pyrimidine-2-yl] - (4-fluoro-phényl) -amine [14] ;
N-[4-(2,4-diméthyl-5-nitro-1H-pyrrole-3-yl)-pyrimidine-2-yl]-N',N'-diméthy1-benzène-1,4-diamine [15] ;
[4-(5-bromo-2,4-diméthyl-1H-pyrrole-3-yl)-pyrimidine-2-yl] - (4-fluoro-phényl) -amine [16] ;
[4-(5-bromo-2,4-diméthyl-1H-pyrrole-3-yl)-pyrimidine-2-yl] - (3-nitro-phényl) -amine [17] ;
[4-(5-chloro-2,4-diméthyl-1H-pyrrole-3-yl)-pyrimidine-2-yl] - (4-fluoro-phényl) -amine [18] ;
[4-(5-diméthylaminométhyl-2,4-diméthyl-1H-pyrrole-3-yl)-pyrimidine-2-yl]-(4-fluoro-phényl) -amine [19] ;
4-[2-(4-diméthylamino-3-nitro-phénylamino)-pyrimidine-4-yl]-3,5-diméthyl-1H-pyrrole-2-carbonitrile [20] ;
N⁴-[4-(2,4-diméthyl-5-nitro-1H-pyrrole-3-yl)-pyrimidine-2-yl]-N',N¹-diméthyl-2-nitro-benzène-1,4-diamine [21] ;
4-[2-(3-fluoro-4-méthyl-phénylamino)-pyrimidine-4-yl]-3,5-diméthyl-1H-pyrrole-2-carbonitrile [22] ;
5-[2-(4-chloro-phénylamino)-pyrimidine-4-yl]-3,4-diméthyl-3H-thiazole-2-one [23] ;
5-[2-(4-méthoxy-phénylamino)-pyrimidine-4-yl]-3,4-diméthyl-3H-thiazole-2-one [24] ;
5-[2-(3-hydroxy-phénylamino)-pyrimidine-4-yl]-3,4-diméthyl-3H-thiazole-2-one [25] ;
5-[2-(4-diméthylamino-phénylamino)-pyrimidine-4-yl)-3,4-diméthyl-3H-thiazole-2-one [26] ;
5-[2-(4-fluoro-3-nitro-phénylamino)-pyrimidine-4-yl]-3,4-diméthyl-3H-thiazole-2-one [28] ;
ester éthylique d'acide 2-chloro-4-[4-(4-méthyl-2-méthyl-amino-thiazole-5-yl)-pyrimidine-2-ylamino]-benzoïque [29] ;
[4-(2-amino-4-méthyl-thiazole-5-yl)-pyrimidine-2-yl]-(3-nitro-phényl)-amine [30] ;
3,4-diméthyl-5-[2-(3-nitro-phénylamino)-pyrimidine-4-yl] -3H-thiazole-2-one [32] ;
5-[2-(4-fluoro-phénylamino)-pyrimidine-4-yl]-3,4-diméthyl-3H-thiazole-2-one [33] ;
5-[2-(4-hydroxy-phénylamino)-pyrimidine-4-yl]-3,4-diméthyl-3H-thiazole-2-one [34] ;
[4-(2-chloro-4-méthyl-thiazole-5-yl)-pyrimidine-2-yl]-(3-nitro-phényl)-amine [35] ;
3,4-diméthyl-5-[2-(4-méthyl-3-nitro-phénylamino)-pyrimidine-4-yl] -3H-thiazole-2-one [36] ;
(4-fluoro-3-méthyl-phényl)-[4-(2-méthoxy-4-méthyl-thiazole-5-yl) -pyrimidine-2-yl] -amine [37] ;
4-[2-(4-diméthylamino-phénylamino)-pyrimidine-4-yl)-1,3,5-triméthyl-1H-pyrrole-2-carbonitrile [38] ; et
4-[2-(4-diméthylamino-3-nitro-phénylamino)-pyrimidine-4-yl)-1,3,5-triméthyl-1H-pyrrole-2-carbonitrile [39].

18. Utilisation suivant la revendication 17, dans laquelle ledit composé est choisi parmi les suivants :
3,5-diméthyl-4-[2-(3-nitro-phénylamino)-pyrimidine-4-yl] -1H-pyrrole-2-carbonitrile [1] ;
4-[2-(4-fluoro-phénylamino)-pyrimidine-4-yl]-3,5-diméthyl-1H-pyrrole-2-carbonitrile [2] ;
4-[2-(3-hydroxy-phénylamino)-pyrimidine-4-yl]-3,5-diméthyl-1H-pyrrole-2-carbonitrile [6] ;
3,5-diméthyl-4-[2-(4-méthyl-3-nitro-phénylamino)-pyrimidine-4-yl]-1H-pyrrole-2-carbonitrile [7] ;
4-[2-(4-fluoro-3-méthyl-phénylamino)-pyrimidine-4-yl]-3,5-diméthyl-1H-pyrrole-2-carbonitrile [11] ;
4-[2-(4-diméthylamino-phénylamino)-pyrimidine-4-yl]-3,5-diméthyl-1H-pyrrole-2-carbonitrile [12] ;
amide d'acide 4-[2-(4-fluoro-phénylamino)-pyrimidine-4-yl)-3,5-diméthyl-1H-pyrrole-2-carboxylique [13] ;
4-[2-(3-fluoro-4-méthyl-phénylamino)-pyrimidine-4-yl]-3,5-diméthyl-1H-pyrrole-2-carbonitrile [22] ;
3,4-diméthyl-5-[2-(3-nitro-phénylamino)-pyrimidine-4-yl]-3H-thiazole-2-one [32];
5-[2-(4-hydroxy-phénylamino)-pyrimidine-4-yl]-3,4-diméthyl-3H-thiazole-2-one [34] ;
3,4-diméthyl-5-[2-(4-méthyl-3-nitro-phénylamino)-pyrimidine-4-yl] -3H-thiazole-2-one [36] ;
5-[2-(4-fluoro-3-méthyl-phénylamino)-pyrimidine-4-yl]-3,4-diméthyl-3H-thiazole-2-one [37] ;
4-[2-(4-diméthylamino-phénylamino)-pyrimidine-4-yl]-1,3,5-triméthyl-1H-pyrrole-2-carbonitrile [38] ; et
4-[2-(4-diméthylamino-3-nitro-phénylamino)-pyrimidine-4-yl]-1,3,5-triméthyl-1H-pyrrole-2-carbonitrile [39].

19. Utilisation suivant la revendication 17, dans laquelle ledit composé est choisi parmi les suivants :
3,5-diméthyl-4-[2-(4-méthyl-3-nitro-phénylamino)-pyrimidine-4-yl]-1H-pyrrole-2-carbonitrile [7] ;
amide d'acide 4-[2-(4-fluoro-phénylamino)-pyrimidine-4-yl]-3,5-diméthyl-1H-pyrrole-2-carboxylique [13] ;
3,4-diméthyl-5-[2-(3-nitro-phénylamino)-pyrimidine-4-yl] -3H-thiazole-2-one [32] ;
3,4-diméthyl-5-[2-(4-méthyl-3-nitro-phénylamino)-pyrimidine-4-yl] -3H-thiazole-2-one [36] ;
5-[2-(4-fluoro-3-méthyl-phénylamino)-pyrimidine-4-yl]-3,4-diméthyl-3H-thiazole-2-one [37] ;
4-[2-(4-diméthylamino-phénylamino)-pyrimidine-4-yl]-1,3,5-triméthyl-1H-pyrrole-2-carbonitrile [38] ; et
4-[2-(4-diméthylamino-3-nitro-phénylamino)-pyrimidine-4-yl]-1,3,5-triméthyl-1H-pyrrole-2-carbonitrile [39].

20. Utilisation suivant l'une quelconque des revendications précédentes, dans laquelle le diabète est le diabète de type II.

21. Utilisation suivant l'une quelconque des revendications précédentes, dans laquelle ledit composé de formule I, ou son sel pharmaceutiquement acceptable, est mélangé à un diluant, excipient ou support pharmaceutiquement acceptable.

22. Utilisation suivant l'une quelconque des revendications précédentes, dans laquelle ledit composé de formule I, est administré en association avec un ou plusieurs autres agents actifs.
